# EUROPÄISCHE PATENTSCHRIFT

(11) **EP 0 501 215 B1**
(45) Veröffentlichungstag und Bekanntmachung des Hinweises auf die Patenterteilung: **24.05.2000**
(21) Anmeldenummer: 92102197.8
(22) Anmeldetag: 10.02.1992
(51) Int. Cl.: C12N 15/62, A61K 47/48, C12N 15/56, C12P 21/08, C07K 16/30

(54) **Monoklonalantikörper, Linker- und Beta-Glucuronidase enthaltende Fusionsproteine zur Prodrug-Aktivierung, ihre Herstellung und Verwendung**
Fusion proteins with monoclonal antibody, Linker and beta Glucuronidase for prodrug activation; preparation and use thereof
Protéines fusionnées anticorps monoclonal-Linker-beta glucuronidase pour l'activation de précurseurs; sa préparation et son utilisation

(30) Priorität: 28.02.1991 DE 4106389
(43) Veröffentlichungstag der Anmeldung: 02.09.1992
(73) Patentinhaber: HOECHST AKTIENGESELLSCHAFT, 65926 Frankfurt am Main (DE)
(72) Erfinder: Seemann, Gerhard, W-3550 Marburg-Elnhausen (DE); Bosslet, Klaus, W-3550 Marburg (DE); Czech, Jörg, W-3550 Marburg (DE); Kolar, Cenek, W-3550 Marburg (DE); Hoffmann, Dieter, W-3550 Marburg-Elnhausen (DE); Sedlacek, Hans-Harald, W-3550 Marburg (DE)

(56) Entgegenhaltungen:
- EP-A- 0 172 045
- EP-A- 0 302 473
- EP-A- 0 352 761
- EP-A- 0 388 914
- EP-A- 0 441 218
- WO-A-88/07378
- WO-A-90/07929
- WO-A-91/08770
- FR-A- 2 591 895
- BIOTECHNOLOGY ABSTRACTS, DERWENT PUBLICATIONS LTD., LONDON, GB ABSTRACT NO. 92-14076 G. SEEMANN ET AL. 'Antibody enzyme fusion protein for antibody directed enzyme prodrug therapy - carcinoembryonic antigen-specific monoclonal antibody and beta-glucuronidase fusion protein'

## Beschreibung

Die Erfindung betrifft Fusionsproteine für die Prodrug-Aktivierung der allgemeinen Formel huTuMAK-L-β-Gluc, wobei huTuMAK für einen humanisierten oder humanen tumorspezifischen monoklonalen Antikörper, ein Fragment oder ein Derivat davon steht, L einen Linker bedeutet und β-Gluc humane β-Glucuronidase beinhaltet. Diese Fusionsproteine werden gentechnisch hergestellt. huTuMAK sorgt für die spezifische Lokalisation von Tumoren, L verbindet huTuMAK mit β-Gluc in einer Weise, welche die spezifischen Eigenschaften beider Fusionspartner nicht behindert und β-Gluc aktiviert eine geeignete Prodrug-Verbindung durch Abspaltung von Glucuronsäure, wobei durch die humanisierten bzw. humanen Fusionspartner ein nahezu autologes System zur Anwendung im Menschen bereitsteht.

Die Kombination von Prodrug und tumorspezifischen Antikörper-Enzym-Konjugaten zur Anwendung als therapeutische Mittel ist in der Fachliteratur beschrieben. Hierbei wurden gegen bestimmtes Gewebe gerichtete Antikörper, an die ein Prodrug-spaltendes Enzym kovalent gebunden sind, einem Tier, welches das transplantierte Gewebe enthält, injiziert, und anschließend wird eine Enzym-aktivierbare Prodrug-Verbindung verabreicht. Unter der Einwirkung des am Gewebe verankerten Antikörper-Enzym-Konjugates wird die Prodrug-Verbindung zum Zellgift umgewandelt, das eine zytotoxische Wirkung gegen das transplantierte Gewebe ausübt.

In WO 88/07378 ist ein zwei Komponenten enthaltendes therapeutisches System, bestehend aus einer Antikörper-Enzym-Komponente beschrieben. Zur Herstellung der Antikörper-Enzym-Konjugate wird hierbei die Verwendung von Nicht-Säuger-Enzymen beschrieben und die von endogenen Enzymen aufgrund der unspezifischen Wirkstoff-Freisetzung ausgeschlossen. Da die exogenen Enzyme von dem Organismus als Fremdantigene erkannt werden, ist deren Verwendung mit dem Nachteil einer Immunantwort gegenüber diesen nicht körpereigenen Stoffen verbunden, aufgrund deren das am Antikörper immobilisierte Enzym desaktiviert und gegebenenfalls das ganze Konjugat eliminiert wird. Weiterhin werden hier p-Bis-N-(2-chlorethyl)-aminobenzylglutaminsäure und deren Derivate als Prodrug verwendet, deren chemische Halbwertzeit nur 5,3 bis 16,5 Stunden beträgt. Die chemische Instabilität eine Prodrug-Verbindung ist aufgrund der zu erwartenden Nebenwirkungen von Nachteil.

In EP A2-0 302 473 wird ebenfalls ein zwei Komponenten enthaltendes therapeutisches System beschrieben, in dem das am Tumorgewebe lokalisierte Antikörper-Enzym-Konjugat eine Prodrug-Verbindung zu einem zytotoxischen Wirkstoff spaltet. Die hierbei unter anderem beschriebene kombinierte Verwendung von Etoposid-4'-phosphat und deren Derivaten als Prodrug und antikörperimmobilisierten alkalischen Phosphatasen zur Freisetzung der Etoposide ist aufgrund der starken Präsenz von endogenen alkalischen Phosphatasen im Serum von Nachteil. Wie in DE A1-38 26 562 beschrieben, werden bereits die Etoposid-4'-phosphate alleinig als therapeutisches Antitumormittel angewandt, wobei die im Serum präsenten Phosphatasen das Etoposid aus dem Prodrugs freisetzen.

Die vorliegende Erfindung wird durch die Patentansprüche beschrieben und im folgenden näher erläutert.

huTuMAK's gekoppelt über L an β-Gluc und gentechnisch hergestellt stellen besonders vorteilhaftes, da nahezu autologes System dar. Die katalytische Aktivität der β-Gluc ist im Fusionsprotein bei pH 7.4 (d.h. unter physiologischen Bedingungen) signifikant höher als die des nativen Enzyms, wenn das Fusionsprotein über die V-Region an das Antigen gebunden ist.

Es wurde gefunden, daß eine chemische Modifizierung der Fusionsproteine, insbesondere eine teilweise oder vollständige Oxidation der Kohlenhydratstrukturen vorzugsweise mit einer nachfolgenden reduktiven Aminierung zu einer verlängerten Halbwertszeit führt. Eine enzymatische Behandlung der erfindungsgemäßen Fusionsproteine mit alkalischer Phosphatase aus z.B. Rinderdarm oder E. coli führte im allgemeinen zu keiner signifikanten Verlängerung der Halbwertszeit.

Die vorliegende Erfindung betrifft Fusionsproteine der Formel
huTuMAK-L-β-Gluc (I)
wobei
huTuMAK einen humanisierten oder humanen tumorspezifischen monoklonalen Antikörper oder ein Fragment davon bedeutet, vorzugsweise die in der EP-A1-0 388 914 beschriebenen MAK.
Besonders bevorzugt enthält das Antikörpertragment huTuMAK das humanisierte MAK Fragment mit den V_{L} und V_{H} Genen gemäß Tabelle 3.

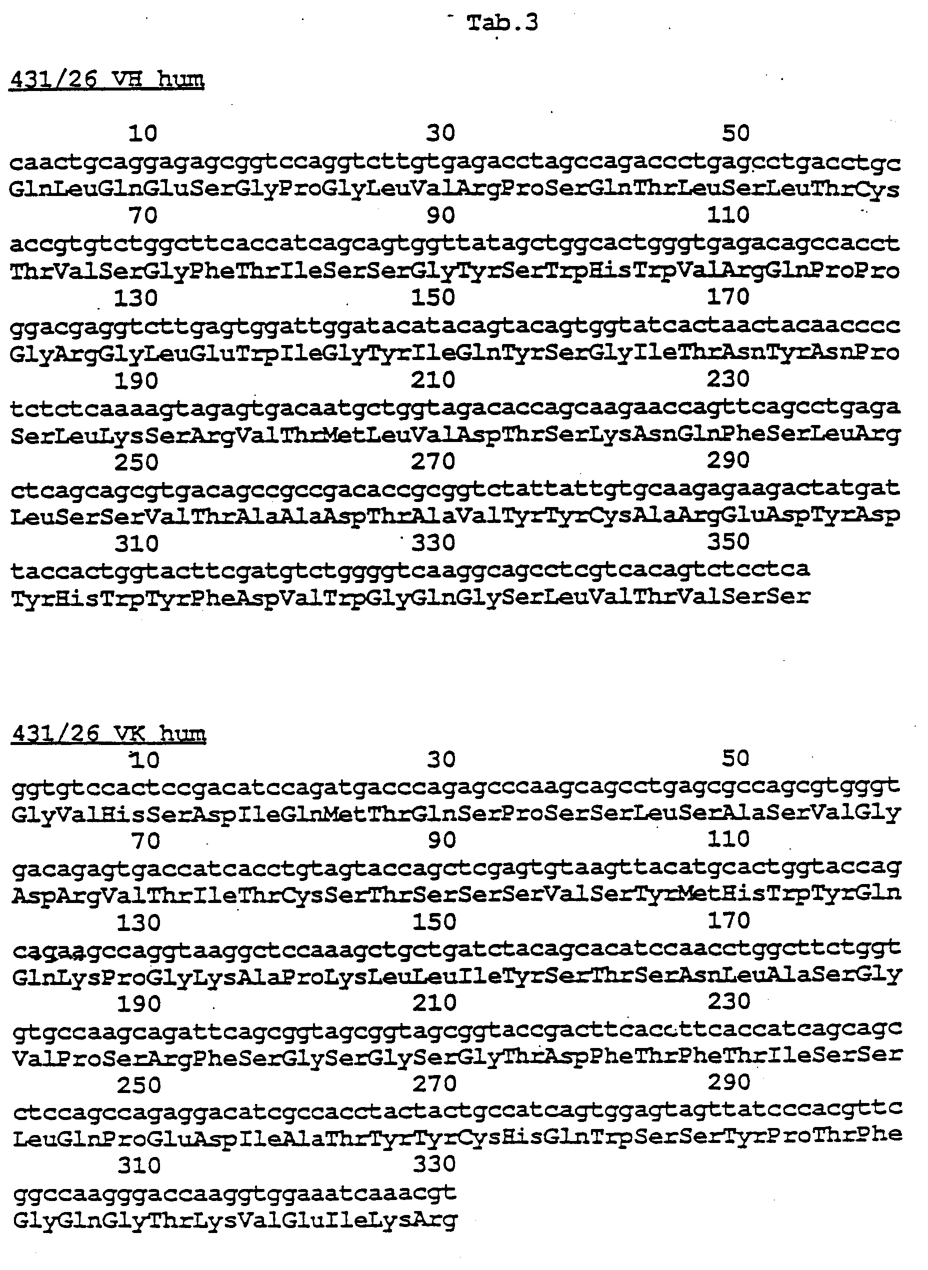

- L: bedeutet Peptid-Linker und enthält eine Hinge-Region eines Immunglobulins, die über eine Peptidsequenz mit dem N-Terminus des reifen Enzyms verbunden ist.
- β-Gluc: bedeutet die vollständige Aminosäuresequenz der humanen β-Glucuronidase bzw. in den zugehörigen Gen-Konstrukten die vollständige cDNA (Oshima A. et al., Proc.Natl.Acad.Sci. USA 84, (1987) 685-689).

Die zur Expression der erfindungsgemäßen Fusionsproteine geeigneten Konstrukte weisen in den Bereichen, die für das Antikörperfragment huTuMAK kodieren, ein CH₁-Exon und mindestens ein Hinge-Exon auf; besonders bevorzugt sind Konstrukte, in denen diese Teile aus einem humanen IgG3 C-Gen stammen. Zur Expression der erfindungsgemäßen Fusionsproteine sind Konstrukte beispielsweise gemäß Beispiel (I) geeignet, wobei die entsprechende leichte Kette des humanisierten TuMAK's coexprimiert wird, um so einen in den Bindungseigenschaften des ursprünglichen TuMAK's möglichst gleichen huTuMAK-Anteil zu erhalten. Die Erfindung betrifft schließlich Verfahren für die gentechnische Herstellung vorgenannter Fusionsproteine, ihre Reinigung sowie ihre Verwendung als Arzneimittel. Fusionsproteine wie beschreiben können zur Prodrug-Aktivierung bei Tumorerkrankungen eingesetzt werden.

In einer weiteren Ausführungsform werden die erfindungsgemäßen Fusionsproteine chemisch modifiziert, um eine verlängerte Halbwertszeit und somit eine verbesserte Lokalisation von Tumoren zu erreichen. Vorzugsweise werden die Fusionsproteine mit einem Oxidationsmittel, z. B. Periodat, behandelt, was im allgemeinen zu einer teilweisen oder vollständigen Spaltung der Kohlenhydratringe und somit zu einer Veränderung der Kohlenhydratstruktur führt. Diese Veränderung führt im allgemeinen zu einer verlängerten Halbwertszeit. Weiterhin ist es vorteilhaft in einem zweiten Reaktionsschritt bestehende Aldehydgruppen zu derivatisieren, beispielsweise durch reduktive Aminierung. Die teilweise oder vollständige Zerstörung der Aldehydgruppen führt im allgemeinen zu einer Reduzierung möglicher Nebenreaktionen mit beispielsweise Plasmaproteinen.
Demzufolge ist es vorteilhaft, die erfindungsgemäßen Fusionsproteine in einen ersten Reaktionsschritt beispielsweise mit Periodat zu oxidieren und in einem zweiten Reaktionsschritt beispielsweise mit Ethanolamin und Cyanoborhydrid reduktiv zu aminieren.

Die nachfolgenden Beispiele beschreiben die gentechnische Synthese eines besonders bevorzugten erfindungsgemäßen Fusionsproteins, dessen Derivatisierung sowie den Nachweis der Funktionsfähigkeit beider Fusionspartner.

### Beispiel (A):

Ausgangsmaterial war das Plasmid pGEM4-HUGP13 (Fig. A). pGEM4-HUGP13 enthält ein cDNA Insert, welches die vollständige kodierende Sequenz für das Enzym humane β-Glucuronidase enthält (Oshima et al. a.a.o.). Alle verwendeten Techniken wurden Maniatis et al., Molecular Cloning: A Laboratory Manual, Second Edition, Cold Spring Harbor Laboratory Press, Gold Spring Harbor USA (1989) entnommen.

### Beispiel (B):

Das Plasmid pGEM4-HUGP13 wurde fit den Restriktionsendonukleasen Pst1 und SalI geschnitten und das 342bp lange Pst1/SalI Fragment, welches die NotI Restriktionsschnittstelle trägt, isoliert. Das Pst1/Sal1 Fragment wurde in den Pst1/Sal1 gespaltenen Vektor pTZ (Fig. B.1)
kloniert und der Klan pTZβGlc350 isoliert (Fig. B.2).

### Beispiel (C):

Der Plasmidklon pTZβGlc350 wurde mit Pst1 gespalten und das doppelsträngige DNA-Fragment βGlc Linker, welches aus den Oligonukleotiden βGlc Linker 1 und βGlc Linker 2 besteht (Tab. 1) und kohäsive Pst1 Enden besitzt, in die geöffnete Pst1 Schnittstelle ligiert.

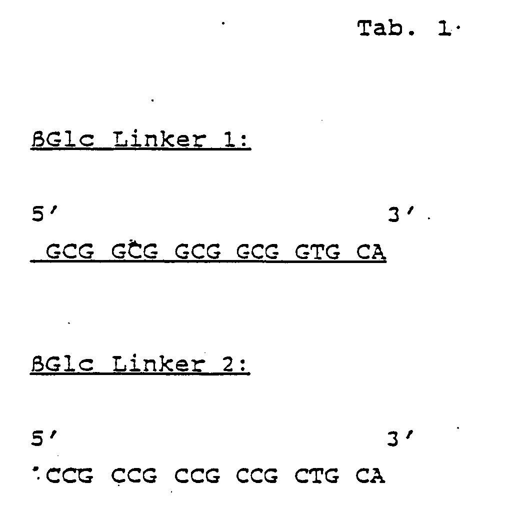

Es wurde der Klon pTZβGlc370 isoliert, bei dem das β-Glucuronidase Fragment an seinem 5' Ende um das DNA-Fragment βGlc Linker verlängert ist und das die vorher vorhandene PST I Schnittstelle verloren, dafür aber an seinem 5' Ende eine neue Pst I Schnittstelle erhalten hat.

### Beispiel (D):

Der Plasmidklon pTZβGlc370 wurde mit Pst I gespalten und mit dem aus den Hinge-Oligonukleotiden 1 und 2b bestehenden Hinge-Linker-Fragment (Tab. 2), welches zwei kohäsive Pst I Enden besitzt, ligiert. Dabei wird die Pst I Schnittstelle am 5' Endes des βGlc370 Fragmentes zerstört. Es wurde der Plasmidklon pTZβGlc420 isoliert, bei dem das βGlc Insert am 5' Ende um das Hinge-Linker-Fragment H verlängert ist (Fig. D).

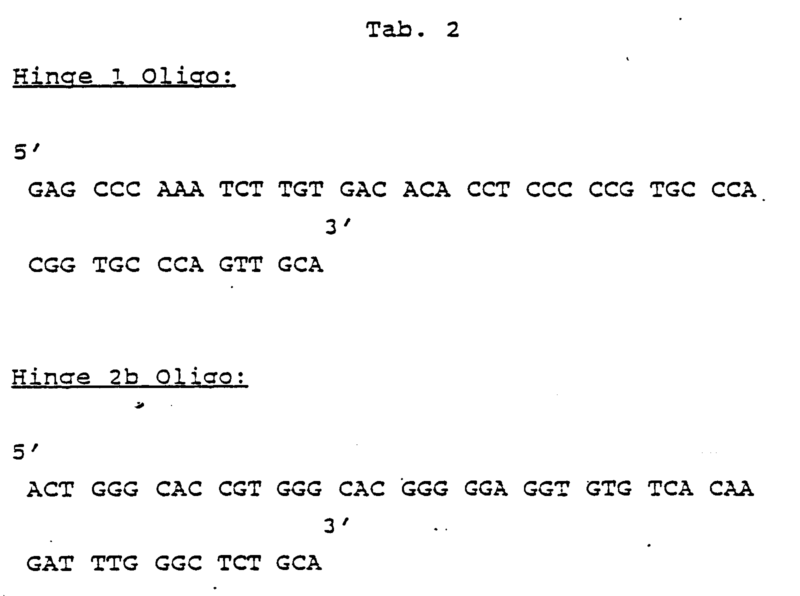

### Beispiel (E):

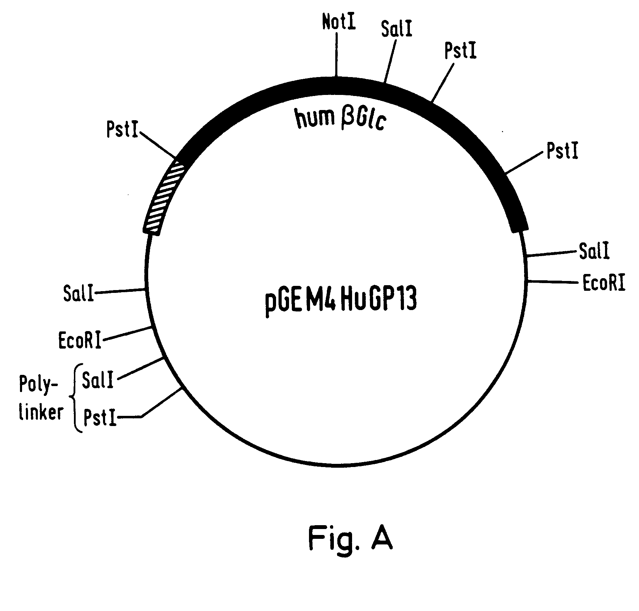

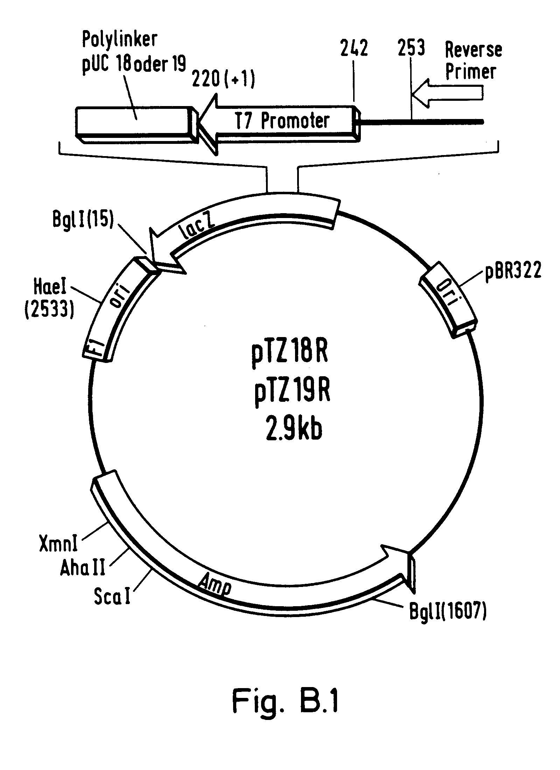

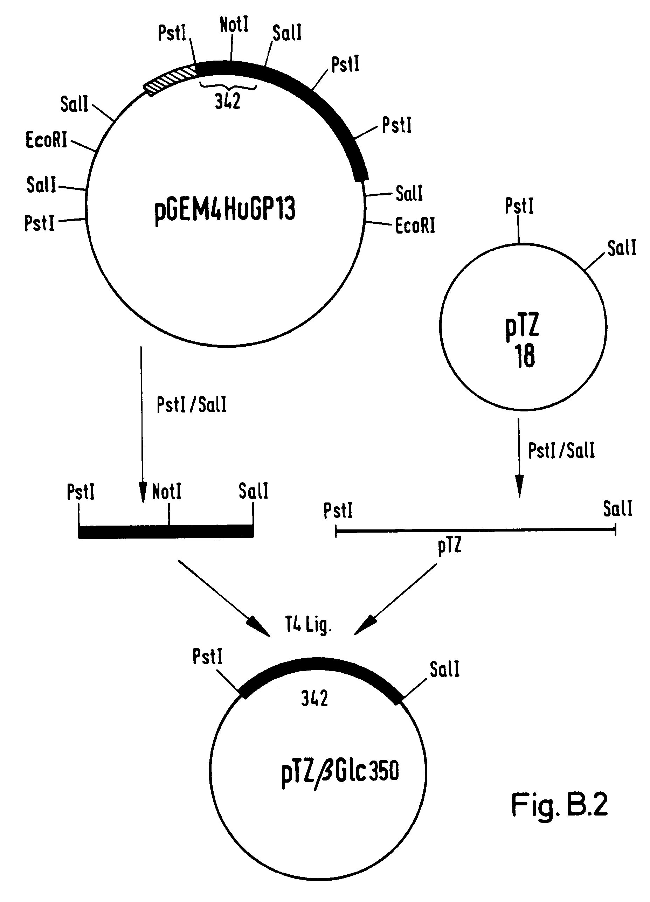

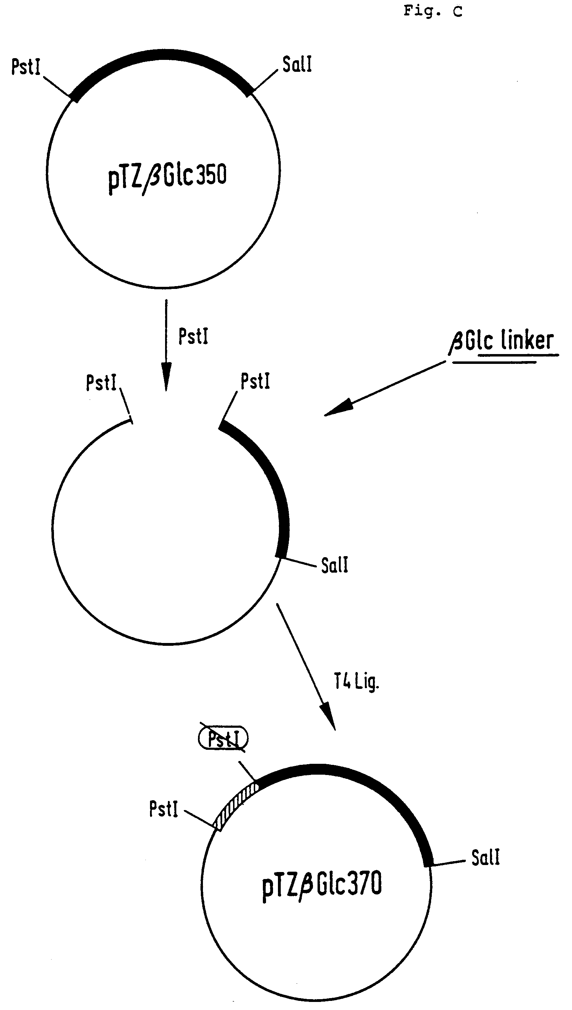

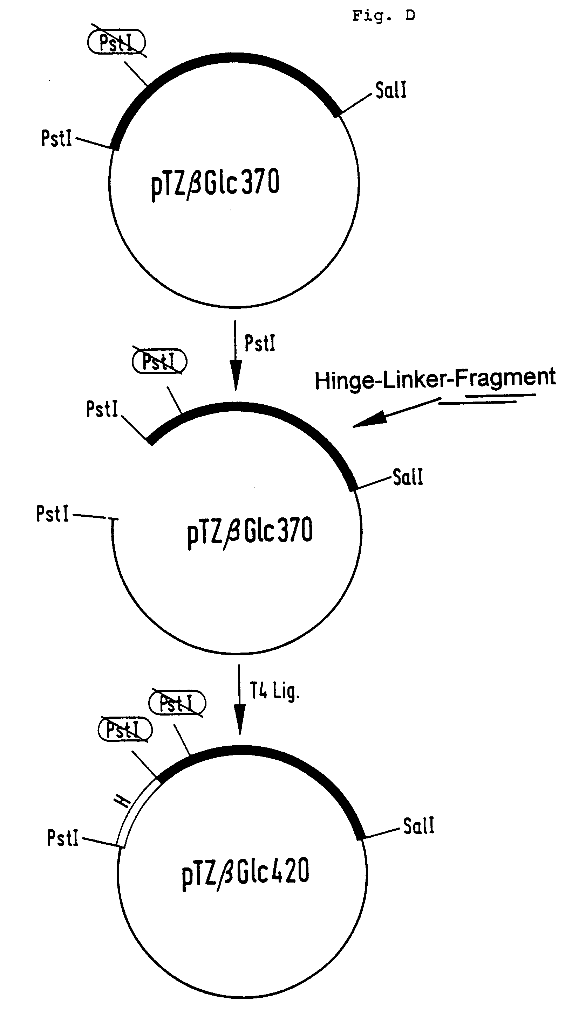

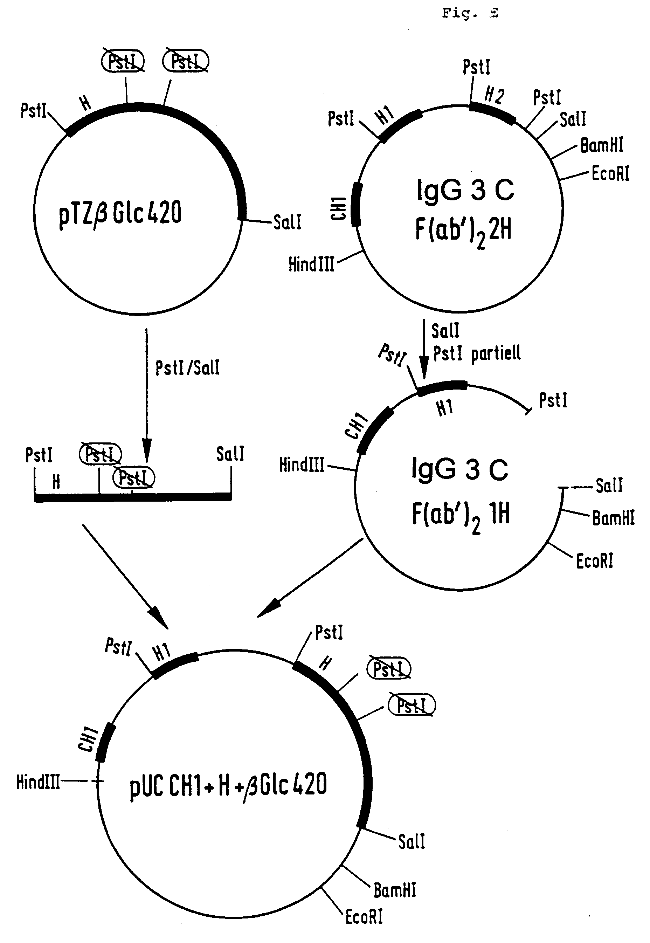

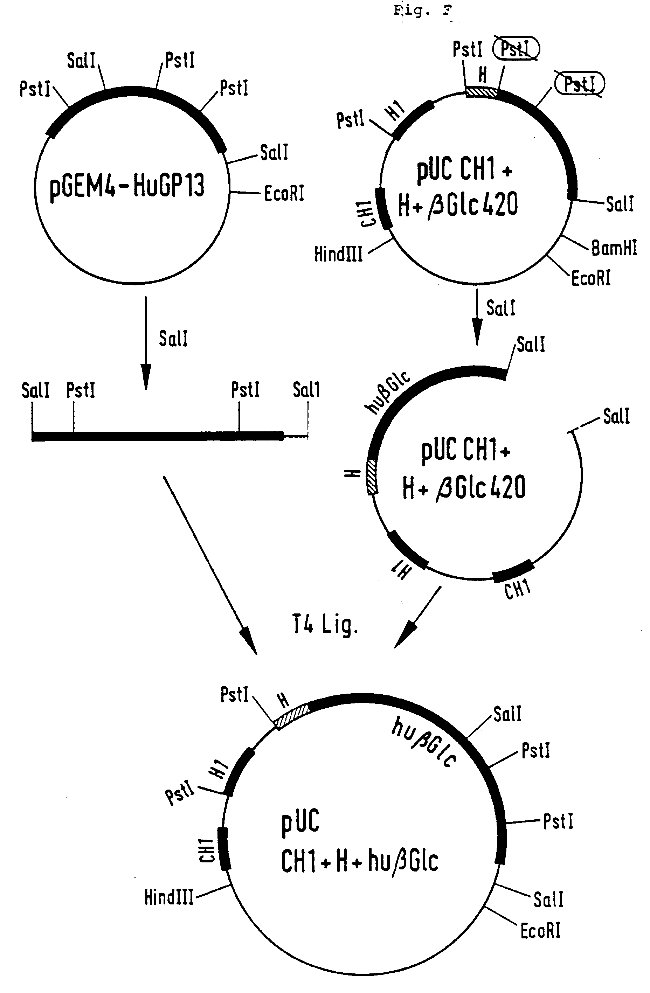

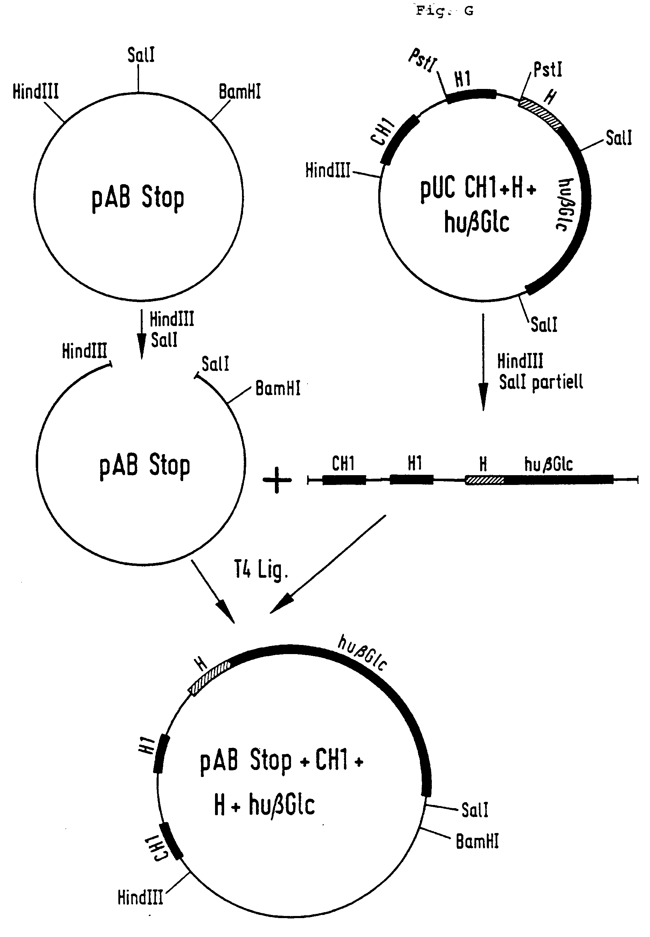

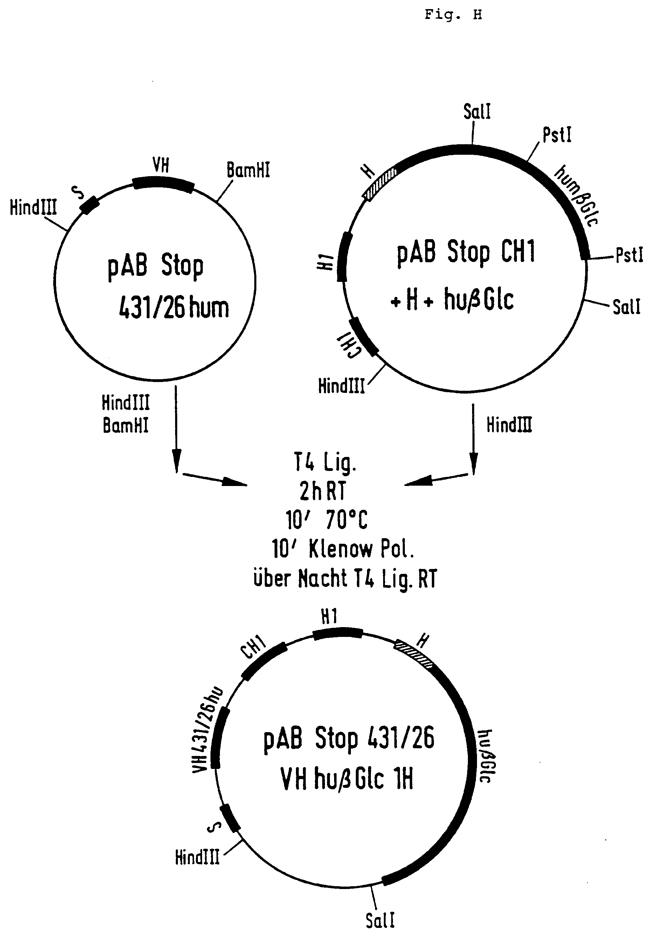

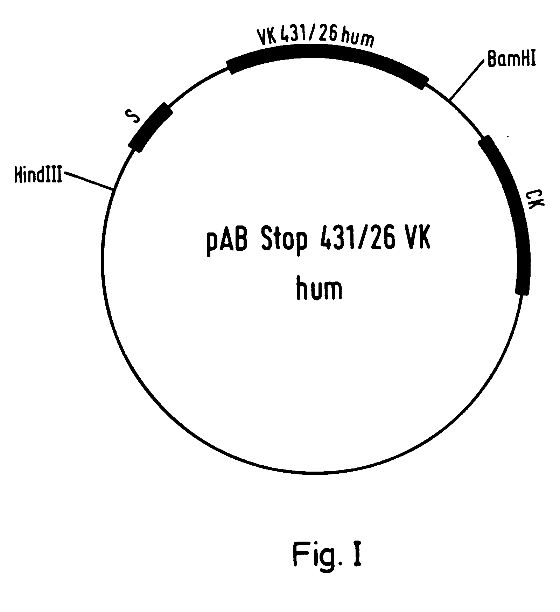

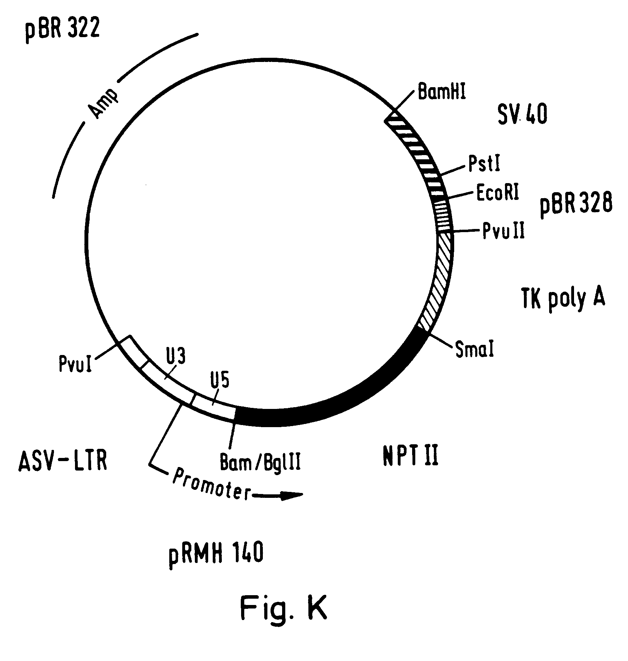

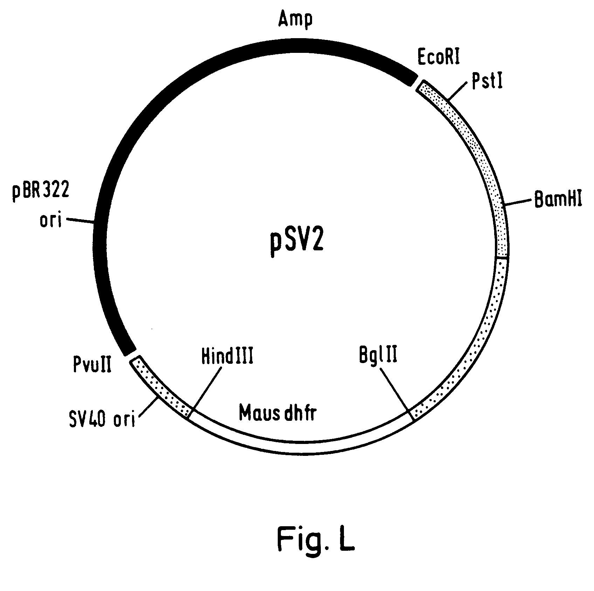

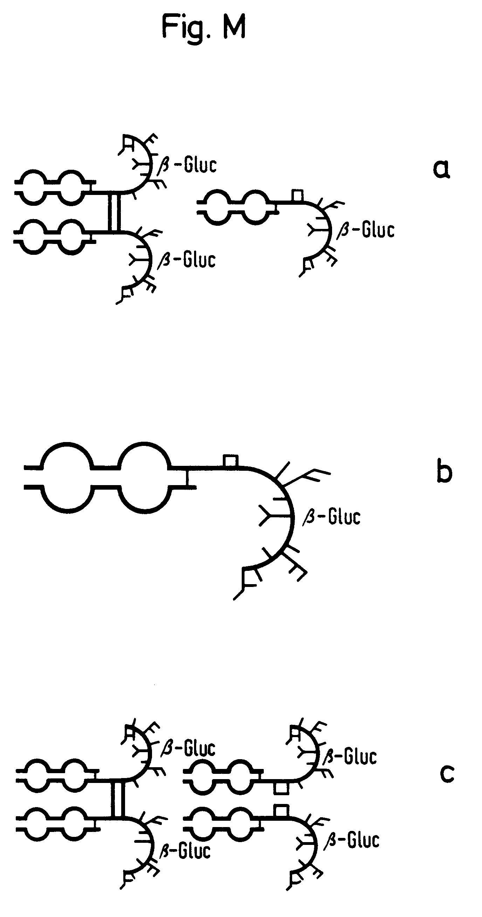

Das Plasmid pTZβGlc420 wurde mit Pst1 und Sal1 gespalten und das 420bp Insert isoliert. Das Plasmid IgG3c F(ab')₂ 2H (EP-A2-O 352 761, Fig. 3, ibidem), welches das CH₁ Exon und zwei Hinge Exons eines humanen IgG3 C-Gens enthält, wurde vollständig mit Sal1 und partiell mit Pst1 gespalten. Mit diesem Sall/Pst1(part.) gespaltenen Plasmid wurde das isolierte 42obp Insert ligiert und der Plasmidklon isoliert, der das CH₁ Exon, ein Hinge Exon und das βGlc420 Fragment enthält, zwischen CH₁ Exon und β-Glucuronidase also die genetische Information zweier Hinge Exons trägt (pUC CH₁ + H + βGlc420) (Fig. E).

### Beispiel (F):

Das Plasmid pGEM4-HUGP13βGlc wurde mit Sall gespalten und das 1750bp Sall-Fragment aus der β-Glucuronidase cDNA isoliert. Das isolierte 1750bp Sall-Fragment wurde mit dem Sall-gespaltenen Plasmid pUC CH₁ + H + βGlc420 ligiert. Es wurde der Plasmidklon pUC CH₁/βGluc isoliert, der ein Fusionsgen aus einem CH₁ Exon, einem Hinge Exon und einem Fusionsexon zwischen einem Hinge Exon und der humanen β-Glucuronidase cDNA enthält (Fig. F).

### Beispiel (G):

Der Expressionsvektor pABstop (Fig. I.1) wurde mit HindIII und Sal1 gespalten. Das Plasmid pUC CH₁ + H + HuβGlc wurde mit HindIII vollständig und mit Sal1 partiell gespalten und das CH₁ + H + huβGlc Insert isoliert. Das CH₁ + H + huβGlc Insert wurde mit dem HindIII/ Sal1-gespaltenen pABstop ligiert und der Klon pABstop CH₁ + H + huβGlc isoliert (Fig. G).

### Beispiel (H):

Der pABstop Vektor pABstop BW 431/26 hum V_{H}, der die humanisierte Version des V_{H}-Gens des anti CEA MAK BW 431/26 (Bosslet K. et al., Eur.J.Nucl.Med. 14, (1988) 523-528) enthält (Sequenzen des humanisierten V_{H}- und V_{K}-Gens siehe Tab. 3), wurde mit HindIII und BamH1 gespalten und das Insert, welches das Signalexon und das V_{H}-Exon enthält, isoliert. Der Plasmidklon pABStop CH₁ + H + huβGlc wurde mit HindIII gespalten und mit den HindIII/BamH1 431/26 hum V_{H}-Fragment ligiert. Nach 2 h Ligation bei Raumtemperatur wurde die Ligation durch 10' Inkubation bei 70° C gestoppt, die noch freien Enden mit Klenow Polymerase und dNTPs aufgefüllt. Dann wurde über Nacht weiterligiert. Nach Transformation wurde mit Hilfe von Restriktionskartierung und Nukleinsäuresequenzanalyse der Klon βABStop 431/26 hum V_{H} huβGlc1H isoliert, der ein Immunglobulin F(ab')₂ Gen mit einem Hinge-Exon enthält, das mit dem kodierenden Bereich der humanen β-Glucuronidase fusioniert ist (Fig. H).

### Beispiel (I):

Der Klon pABStop 431/26 hum V_{H} huβGlc1H wurde zusammen mit einem Plasmidklon, der die leichte Kette des humanisierten BW 431/26 trägt (Fig. I) und zwei Plasmiden, die ein Neomycin- (Fig. K) bzw. ein Methotrexat-Resistenzgen (Fig. L) tragen, in BHK Zellen transfiziert. Es wurde ein Fusionsprotein ausgeprägt, das sowohl die Antigenbindungseigenschaften des MAK BW 431/26hum als auch die enzymatische Aktivität der humanen β-Glucuronidase hat.

### Beispiel J:

### Nachweis der Antigenbindungseigenschaften und der enzymatischen Aktivität des 431/26hum V_{H}huβGlc 1H Fusionsproteins

Die Fähigkeit des 431/26hum V_{H}huβGlc 1H Fusionsproteins spezifisch an das durch den 431/26 definierte Epitop auf CEA (Carcinoembryonic Antigen) zu binden und gleichzeitig die enzymatische Aktivität der humanen β-Glucuronidase auszuüben, wurde in einem Spezifitäts-Enzymaktivitätstest bestimmt. Dieser Test wird wie im folgenden
beschrieben durchgeführt:
- Polystyrol (96 well) Mikrotitrationsplatten (U-Form, Typ B, Fa. Nunc, Best.-Nr. 4-60445) werden mit gereinigtem CEA (1-5 µg CEA/ml, hiervon 75 µl/Vertiefung) oder mit GIT-Mucin (gleiche Menge wie CEA) über Nacht bei R.T. inkubiert.
- Das nicht adsorbierte Antigen wird abgesaugt und 3x mit 0.05 M Tris-Citrat-Puffer, pH 7.4, gewaschen.
- Die Mikrotitrationsplatten werden über Nacht mit der Öffnung nach unten auf Zellstoff bei R.T. stehen gelassen.
- Die Mikrotitrationsplatten werden 30 Minuten bei 20°C mit 250 µl 1%iger Caseinlösung in PBS, pH 7.2 /well inkubiert (Blockierungslösung).
- Während des Blockierens wird das Substrat angesetzt. Die Menge des Substrates richtet sich nach der Zahl der zu testenden Überstände. Das Substrat wird für jeden Test frisch angesetzt.
   Substrat: 4-Methylumbelliferyl β-D-Glucuronide (Best.-Nr.: M-9130 bei Fa. Sigma), 2.5 mM in 200 mM NaAcetatpuffer, pH 5.0, mit 0.01 % BSA
- Die Blockierungslösung wird abgesaugt und je 50 µl BHK-Zellüberstand, welcher das Fusionsprotein enthält, wird auf die mit CEA- bzw. GIT-Mucin gecoatete Mikrotitrationsplatte aufgetragen (benötigtes Probenvolumen also mindestens 120 µl).
- Anschlieβend wird 30 Minuten bei R.T. inkubiert.
- Die Platten werden 3x mit ELISA-Waschpuffer (Behring, OSEW 96) gewaschen.
- Das Substrat wird aufgetragen (50 µl/well) und 2 Stunden bei 37° C inkuhiert. Wegen möglicher Verdunstung wird die Platte abgedeckt.
- Nach 2 Stunden werden 150 µl Stopplösung/well pipettiert (Stopplösung = 0,2 M Glycin + 0,2 % SDS, pH 11.7).
- Die Auswertung kann nun unter einer UV-Lampe (Anregungsenergie 380 nm) oder in einem Fluoreszenzmessgerät. (Fluoroscan II, ICM- Biomedicals, Kat-Nr.: 78-611-00) erfolgen.

Mittels dieses Spezifitäts-Enzymaktivitätstests konnte gezeigt werden, daß in den mit CEA beschichteten Vertiefungen ("wells") fluoreszierendes 4-Methylumbelliferol nachzuweisen war, wenn die Enzymaktivität bei pH 5, dem katalytischen Optimum, bestimmt wurde (Tab.4).

**Tabelle 4**

| **Ausverdünnung von Fusionsproteinzellkulturüberstand (B73/2) auf CEA und GIT Mucin** | | | |
|---|---|---|---|
| | Substrat in unterschiedlichen Lösungen | | |
| Verdünnungsstufen | 0.2 M Natriumacetatpuffer + 0.01 % BSA, pH 5, auf CEA | PBS, pH 7.2 auf CEA | PBS,pH 7.2 auf GIT Mucin |
| konzentriert | 9118 | 2725 | 115,7 |
| 1:2 | 7678 | 2141 | 93,37 |
| 1:4 | 4662 | 1195 | 73,39 |
| 1:8 | 2927 | 618,5 | 60,68 |
| 1:16 | 1657 | 332,1 | 53,69 |
| 1:32 | 853 | 168,2 | 40,44 |
| 1:64 | 425 | 99,26 | 48,21 |
| 1:128 | 192,5 | 57,89 | 47,48 |

Eine Bestimmung der Umsatzrate bei pH 7.2 ergab, daß das Fusionsprotein bei diesem physiologischen pH noch ≈ 25 % der Umsatzrate bei pH 5 zeigte. Auf den mit GIT-Mucin gecoateten und bei pH 5 vermessenen Negativkontrollplatten konnte keine signifikante Methylumbelliferolfreisetzung gemessen werden.
Dieser Befund zeigt, daß die humanisierte V-Region des 431/26hum V_{H}hußGlc 1H Fusionsproteins ihre Epitopspezifität behalten hat und der β-Glucuronidaseanteil des Fusionsproteins in der Lage ist, ähnlich wie das menschliche native Enzym, das β-Glucuronid des 4-Methylumbelliferols zu spalten.

### Beispiel K:

### Nachweis der funktionellen Identität der V-Region des 431/26hum V_{H}huβGlc 1H Fusionsproteins mit der des humanisierten MAK BW 431/26 und der des murinen MAK BW 431/26

Im Beispiel J wurde gezeigt, daß das 431/26hum V_{H}huβGlc 1H Fusionsprotein ein gewisses CEA-Bindungspotential sowie β-Glucuronidaseaktivität besitzt. Der im folgenden beschriebene antigenspezifische Kompetitionstest macht eine Aussage über die Identität der CEA-Epitope, die von den kompetitierenden Molekülen erkannt werden sowie über die Interaktionsstärke der Epitop-Fusionsprotein- bzw. Epitop-Antikörper-Reaktion.

Dieser Test wird wie im folgenden beschrieben durchgeführt:
- Polystyrol 96 well Mikrotitrationsplatten (U-Form, Typ B, Fa. Nunc, gest.-Nr.: 4-60445) werden mit gereinigtem CEA (1-5 µg CEA/ml, hiervon 75 µl/well) oder mit GIT-Mucin (gleiche Menge wie CEA) über Nacht bei R.T. inkubiert.
- Das nicht adsorbierte Antigen wird abgesaugt und 3x mit 0.05 M Tris-Citrat-Puffer, pH 7.4, gewaschen.
- Die Mikrotitrationsplatten werden über Nacht mit der Öffnung nach unten auf Zellstoff bei R.T. stehen gelassen.
- Die Mikrotitrationsplatten werden 30 Minuten bei R.T. mit 250 µl 1%iger Caseinlösung in PBS, pH 7.2 /well inkubiert (Blockierungslösung).
- 50 µl des MAk BW 431/26 in einer Konzentration von 5 ng/ml werden mit 50 µl 10fach konzentriertem Überstand des humanisierten MAk BW 431/26 bzw. des Fusionsproteins sowie serieller 2x-Verdünnungen gemischt.
- Von diesen Mischungen werden 50 µl Aliquots in die wells der mit CEA- bzw. GIT-Mucin gecoateten Mikrotitrationsplatten pipettiert.
- Die Mikrotitrationsplatten werden für 30 Minuten bei R.T. inkubiert.
- Anschließend werden die Platten 3x mit ELISA-Waschpuffer (Fa. Behringwerke AG, Best.-Nr. OSEW 96, 250 µl) gewaschen.
- Anschließend werden 50 µl eines 1:250 verdünnten Ziege anti Maus Ig Antikörpers, welcher mit alkalischer Phosphatase gekoppelt ist (Southern Biotechnology Associates, Best.-Nr.: 1010-04), zugegeben.
- Nach 30 Minuten Inkubation bei R.T. und 3maligem Waschen wird die Substratreaktion wie folgt durchgeführt:
- 30 µl pro well NADP 0,1 mM zugeben (7,65 mg lösen in 100 ml 20 mM Tris; 0,1 mM MgSO₄, pH 9.5); die Lösung kann bei -20° C mehrere Monate gelagert werden.
- 30 Minuten bei R.T. inkubieren.
- Während der Inkubation mit NADP das Verstärkersystem ansetzen: (5 ml pro Platte)
   2 Teile INT (2,5 mg/ml mit 30%igem Ethanol im Ultraschallbad lösen; immer frisch ansetzen)
   + 1 Teil PBS, pH 7.2
   + 1 Teil Diaphorase (1 mg/ml PBS, pH 7.2)
   + 1 Teil ADH (0,5 mg/ml PBS, pH 7.2)
- 50 µl des Verstärkersystems zugeben
- bei gewünschter Reaktionsstärke die Reaktion mit 0,1 N H₂SO₄ 100 µl pro well abstoppen
- bei 492 nm im TITERTEK^{R} MULTISCAN messen (Blankwert = 50 µl NADP + 50 µl Verstärkerlösung + 100 µl 0,1 H₂SO₄)

NADP - Fa. Sigma, Best.-Nr. N-0505
INT - Fa. Sigma, Best.-Nr. I-8377
ADH - Fa. Sigma, Best.-Nr. A-3263
Diaphorase - Fa. Sigma, Best.-Nr. D-2381

In diesem antigenspezifischen Kompetitionstest bedeutet die Reduktion der Extinktion eine Kompetition der miteinander kompetitierenden Moleküle um das gleiche oder räumlich sehr nahe zusammenliegende Epitope.

Die erhaltenen Inhibitionsdaten zeigen, daß sowohl das Fusionsprotein 431/26hum V_{H}huβGlc 1H als auch der humanisierte MAK 431/26 die Bindung des murinen MAK BW 431/26 an sein CEA-Epitop blockieren. Eine Inhibition von 50 % wird bei einem 200molaren Überschuß der entsprechenden Kompetitoren erreicht. Hieraus ist zu folgern, daß die Avidität des Fusionsproteins zu dem CEA-Epitop vergleichbar ist mit der des humanisierten MAK 431/26. Desweiteren bindet das Fusionsprotein und der humanisierte MAK an das gleiche bzw. ein räumlich dem durch den murinen MAK BW 431/26 auf CEA definierten Epitop sehr nahe liegendes Epitop.

### Beispiel L:

### Nachweis der Gewebespezifität des 431/26hum V_{H}huβGlc 2H Fusionsproteins

In Beispiel J wurde unter anderem gezeigt, daß das 431/26hum V_{H}huβGlc 1H Fusionsprotein an gereinigtes CEA binden kann.

In Beispiel K wurde gezeigt, daß die V-Region des Fusionsproteins in der Lage ist, mit der V-Region des murinen BW 431/26 um das gleiche oder ein sehr naheliegendes Epitop zu kompetitieren. Mit Hilfe des im folgenden beschriebenen indirekten für β-Glucuronidase spezifischen immunhistochemischen Tests kann die Feinspezifität des Fusionsproteins auf kryopräservierten Geweben ermittelt werden.

Der Test ist im folgenden beschrieben:
- 6 µm dicke Gefrierschnitte werden auf Objektträger aufgezogen und mindestens 30 Minuten luftgetrocknet.
- Anschließend werden die Objektträger 10 Sekunden in -20° C kaltem Aceton fixiert.
- Die Objektträger werden 5 Minuten in Tris/NaCl-Waschpuffer, pH 7.4, mit 0,1 % BSA gewaschen.
- 20-100 µl Fusionsprotein enthaltender BHK-Zellüberstand wird (konzentriert oder verdünnt in Tris/BSA, pH 7.4) pro Schnitt aufgetragen und 30 Minuten in einer feuchten Kammer bei R.T. inkubiert.
- Die Objektträger werden 5 Minuten in Tris/Nacl-Waschpuffer, pH 7.4, mit 0,1 % BSA gewaschen.
- Pro Schnitt werden 50 µl Hybridomüberstand des murinen anti β-Glucuronidase MAK BW 2118/157 hinzugegeben und die Objektträger für 30 Minuten in einer feuchten Kammer bei R.T. inkubiert.
- Anschließend werden die Objektträger 5 Minuten in Tris/NaCl- Waschpuffer, pH 7.4, mit 0,1 % BSA gewaschen.
- Es werden 20-100 µl Brücken-AK (Kaninchen-anti Maus Igc 1:100 verd. in Human-Serum, pH 7.4) pro Schnitt aufgetragen und 30 Minuten in einer feuchten Kammer bei R.T. inkubiert.
- Anschließend werden die Objektträger 5 Minuten in Tris/NaCl-Waschpuffer, pH 7.4, mit 0,1 % BSA gewaschen.
- Danach werden 20-100 µl APAAP-Komplex (Maus-anti AP 1:100 verd. in Tris/BSA, pH 7.4) pro Schnitt aufgetragen und 30 Minuten in einer feuchten Kammer bei R.T. inkubiert.
- Anschließend werden die Objektträger 5 Minuten in Tris/NaCl- Waschpuffer, pH 7.4, mit 0,1 % BSA gewaschen.
- Das Substrat für die alkalische Phosphatase wird wie folgt angesetzt (100 ml Substratlösung, ausreichend für eine Glasküvette):

| | | |
|---|---|---|
| Lösung 1: | 3,7 g | NaCl |
| | 2,7 g | Tris/Base in 75 ml Aqua dest lösen |
| | + 26,8 ml | Propandiol-Puffer, pH 9.75, einstellen mit HCl |
| | + 42,9 mg | Levamisol => klare, farblose Lösung |
| Lösung 2: | 21,4 mg | Natriumnitrit in 535 µl Aqua dest lösen |
| | | => klare, farblose Lösung |
| Lösung 3: | 53,5 mg | Naphthol-AS-Biphosphat in 642 µl Dimethylformamid (DMF) lösen => klare, gelbliche Lösung |

- In Lösung 2 (Natriumnitrit) 368 µl 5%ige Neufuchsin-Lsg. geben und 1 Minute (Stoppuhr) reagieren lassen, so daß sich eine klare, braune Lösung ergibt
- Lösung 2 (Natriumnitrit mit Neufuchsin) und Lösung 3 (Naphthol-AS-Biphosphat) in Lösung 1 (Tris/Nacl/Propandiol-Puffer) geben => klare, gelbliche Lösung

- pH 8.8 einstellen mit HCl => trübe, gelbliche Lösung
- Lösung filtrieren und auf die Objektträger geben und 15 Minuten auf einem Schüttler reagieren lassen => Lösung wird trüb.
- Objektträger 10 Minuten in Tris/NaCl-Puffer, pH 7.4, waschen
- Objektträger 10 Minuten in Aqua dest waschen
- Nach 2 Stunden Lufttrocknen werden die Objektträger in Kaiser's Glyzeringelatine bei 56° C eingedeckelt.

Die spezifische Bindung des Fusionsproteins wurde lichtmikroskopisch durch die Rotfärbung der Epitoppositiven Gewebeschnitte nachgewiesen. Vergleichende Untersuchungen mit dem murinen MAK BW 431/26, der mittels der indirekten APAAP-Technik nachgewiesen wurde (Cordell et al., J. Histochem. Cytochem. 32, 219, 1984), ergaben, daß die Gewebespezifität des Fusionsproteins mit der des murinen MAK BW 431/26 genau übereinstimmte, d.h., daß sowohl der Reaktionstyp im individuellen Präparat, wie auch die Anzahl der positiven und negativen Befunde aus einer Vielzahl verschiedener Karzinome und Normalgewebe identisch ist.

### Beispiel M:

### Reinigung des 431/26hum V_{H}huβGlc 1H Fusionsproteins

Murine bzw. humanisierte MAK können mittels immunaffinitätschromatographischer Verfahren, die für den Fc-Teil dieser Moleküle selektiv sind, gereinigt werden. Da in dem 431/26hum V_{H}huβGlc 1H Fusionsprotein kein Fc-Teil vorhanden ist, mußte eine alternative hochselektive immunaffinitätschromatographische Methode entwickelt werden. Neben der Selektivität dieser zu entwickelnden Methode müssen die Isolationsbedingungen sehr milde sein, um die im sauren wie auch alkalischen Bereich sehr labile β-Glucuronidase nicht zu schädigen.

Das Prinzip der Methode besteht in der Reinigung des Fusionsproteins aus Überständen transfizierter BHK-Zellen, mittels eines gegen die humanisierte V-Region gerichteten anti-idiotypischen MAK. Die Herstellung solcher MAK ist literaturbekannt (Walter et al., Behring Inst. Mitt., 82, 182-192, 1988). Dieser anti-idiotypische MAK kann sowohl murin wie auch humanisiert sein. Der MAK ist vorzugsweise auf einer festen Phase so immobilisiert, daß seine V-Region nicht geschädigt wurde. Beispiele hierzu sind literaturbekannt (Fleminger et al., Applied Biochem. Biotechnol., 23, 123-137, 1990; Horsfall et al., JIM 104, 43-49, 1987).

Der so auf der festen Phase nach bekannten Methoden immobilisierte anti-idiotypische MAK bindet das aus transfizierten BHK-Zellen zu reinigende Fusionsprotein sehr effizient, z.B. bei pH 7, hat aber die überraschende Eigenschaft, daß er das Fusionsprotein schon bei einer Absenkung des pH-Wertes um 2,5 Stufen, auf pH 5.5, nicht mehr bindet. Das Fusionsprotein wird durch diese milde pH-Elutionstechnik weder in seiner Fähigkeit, an CEA zu binden, noch in seiner enzymatischen Aktivität beeinträchtigt (Methodik siehe Beispiel J).
In Tab. 5 sind die OD Werte und fluorogenen Einheiten (FU) der einzelnen Fraktionen aus einer Reinigung mit Hilfe des Festphasen-immobilisierten, anti-idiotypischen MAK BW 2064/34 gezeigt.

**Tabelle 5**

| **Anti-idiotype Affinitätschromatographie** | | | | |
|---|---|---|---|---|
| Fraktionen | OD in % | FU in % | pH-wert | Chromatografieverlauf |
| 1-5 | 1 | 0 | 7.2 | Vorspülen der Säule mit PBS, pH 7.2 |
| 6-142 | 20 | 0 | 7.2 | Probenauftrag |
| 143-162 | 1 | 0 | 7.2 | Spülen der Säule mit PBS, pH 7.2 |
| 163 | 1 | 0 | 7.2 | Elution mit PBS, pH 4.2 |
| 164 | 1 | 0 | 7.2 | |
| 165 | 1 | 0 | 7.2 | |
| 166 | 1 | 0 | 6.8 | |
| 167 | 2 | 10 | 6.1 | |
| 168 | 5 | 20 | 5.7 | |
| 169 | 16 | 40 | 5.6 | |
| 170 | 23 | 80 | 5.5 | |
| 171 | 26 | 100 | 5.4 | |
| 172 | 24 | 80 | 5.3 | |
| 173 | 19 | 60 | 5.2 | |
| 174 | 14 | 40 | 5.2 | |
| 175 | 10 | 30 | 5.1 | |
| 176 | 8 | 25 | 5.1 | |
| 177 | 6 | 20 | 5.1 | |
| 178 | 3 | 10 | 5.0 | |
| 179 | 2 | 5 | 5.0 | |
| 180 | 1 | 0 | 5.0 | |
| 1 Fraktion = 6.6 min sammeln (bei 18 ml/h Pumpengeschwindigkeit) = 2 ml Die FU-Werte sind in % des höchsten Wertes (Fraktion 171) angegeben. | | | | |

Mittels der OD Messung bei 280 nm wurde die Elution des Fusionsproteins als Protein gemessen. Zusätzlich wurden die isolierten Fraktionen auf spezifische Bindung an CEA und gleichzeitige Enzymaktivität im Spezifitätsenzymaktivitätstest überprüft (Beispiel J). Die Werte zeigen, daß die gesamte spezifische Bindungs- und Enzymaktivität in einem Peak (um pH 5.0 bis pH 5.6 Elutionspeak) konzentriert war. Hieraus ist zu folgern, daß die so beschriebene Methode der Antiidiotyp-Affinitätschromatographie eine sehr effiziente und selektive Reinigungstechnik für das 431/26hum V_{H}huβGlc 1H Fusionsprotein darstellt.

### Beispiel N:

### Gelchromatographie der Fusionsproteine

Die überstände von den das 431/26hum V_{H} huβGlc 1H Fusionsprotein sekretierenden BHK Zellen (B 70/6, B 74/2, B 72/72, B 73/2) wurden abgenommen, sterilfiltriert und einer analytischen Gelfiltration unterworfen. Dazu wurde eine TSK G3000 SW-XL Säule (7.8 x 300 mm) mit 0,1 M Na-Phosphatpuffer, pH 6.7, + 0.02 % NaN₃ equilibriert, 20 µl des Überstandes aufgetragen und mit einer Flußrate von 0.6 ml/min eluiert. Beginnend mit einer Elutionszeit von 9 min (Ausschlußvolumen 9.5 min) wurden Fraktionen (je 0.3 min) gesammelt und auf β-Glucuronidaseaktivität getestet.

Dazu wurden 25 µl aus der jeweiligen Fraktion mit 75 µl Substratlösung (2.5 mM 4-Methylumbelliferyl-β-glucuronid in 200 mM Na-Acetatpuffer, pH 5, + 0.1 mg/ml BSA) gemischt und bei 37° C 2 Stunden inkubiert. Dann wurde mit 1.5 ml 0.2 M Glycin/0.2 % SDS-Lösung, pH 11.7, abgestoppt und der von der Glucuronidase freigesetzte fluoreszierende Label mit einem Hitachi Fluorometer (bei 360 nM Anregungswellenlänge, 450 nM Emissionswellenlänge) quantifiziert.

### Ergebnis:

Alle 4 Konstrukte zeigen einen einzigen Hauptaktivitätspeak zwischen Fraktionen 4 und 6 (Tabelle 6). Das entspricht Retentionszeiten von ca. 10.2 - 10.8 min. Die in den Überständen vorliegenden Glucuronidaseaktiven Fusionsproteine haben somit Retentionszeiten, die in der gleichen Gößenordnung wie die von chemisch hergestellten Antikörper-β-Glucuronidasekonstrukten (10.4 min) liegen. Die Retentionszeit für das freie Enzym liegen bei 11.9, für den freien Antikörper bei 12.3 min.

**Tabelle 6**

| **Gelfiltration unterschiedlicher Fusionsproteine** | | | | |
|---|---|---|---|---|
| Inkubation: 25 µl, 37° C, 120 min Substanzkonzentration: 1.875 mM; 0.1 mg/ml BSA Fraktion je 0.3 min; Start bei 9 min freigesetzter Label/Assay (FU) | | | | |

| Fraktionen | B70/6 | B74/2 | B72/72 | B73/2 |
|---|---|---|---|---|
| 1 | 11 | 17 | 15 | 15 |
| 2 | 22 | 35 | 44 | 38 |
| 3 | 125 | 97 | 873 | 1014 |
| 4 | 1072 | 196 | 2959 | 3994 |
| 5 | 1588 | 165 | 2206 | 3141 |
| 6 | 1532 | 120 | 1133 | 1760 |
| 7 | 941 | 103 | 581 | 926 |
| 8 | 710 | 69 | 376 | 723 |
| 9 | 500 | 108 | 302 | 626 |
| 10 | 371 | 123 | 316 | 613 |
| 11 | 320 | 107 | 263 | 472 |
| 12 | 254 | 91 | 210 | 456 |
| 13 | 224 | 57 | 146 | 357 |
| 14 | 190 | 65 | 134 | 332 |
| 15 | 171 | 52 | 83 | 294 |
| 16 | 167 | 44 | 99 | 243 |
| 17 | 100 | 38 | 73 | 217 |
| 18 | 129 | 34 | 55 | 179 |
| 19 | 75 | 45 | 48 | 155 |
| 20 | 66 | 41 | 36 | 129 |
| 21 | | | | 118 |
| 22 | | | | 93 |
| 23 | | | | 78 |
| 24 | | | | 61 |
| 25 | | | | 24 |
| 26 | | | | 51 |

### Beispiel O:

### Molekulare Charakterisierung des 431/26 hum V_{H} huβGlc1H Fusionsproteins

In Beispiel M wurden die Fusionsproteine mittels Antiidiotyp-Affinitätschromatogrpahie gereinigt. Aliquots aus dem Elutionspeak von pH 5.5 wurden in der 10 % SDS-PAGE unter nicht reduzierenden bzw. reduzierenden Bedingungen elektrophoriert und im Westernblot mit Hilfe antiidiotypischer MAK bzw. mit anti β-Glucuronidase MAK immungefärbt (Towbin and Gordon (1979), Proc. Natl. Acad. Sci. USA 76: 4350-4354).

Unter nicht reduzierenden Bedingungen konnte beim 431/26 hum V_{H} huβGlc1H Fusionsprotein eine Hauptbande von ≈ 125 kDa und eine Bande von 250 kDa nachgewiesen werden, die sowohl von anti-idiotypischen MAk als auch von anti β-Glucuronidase MAk im Western Blot nachweisbar waren. Unter reduzierenden Bedingungen war weder durch die anti-idiotypischen noch durch die antiβ-Glucuronidase MAk eine Immunfärbung erkennbar. In der reduzierenden SDS-PAGE konnte eine 100 kDa und eine 25 kDa Bande nachgewiesen werden. Diese unter denaturierenden Bedingungen analysierten Moleküle liegen allerdings unter nativen Bedingungen nach TSK G 3000 SW-XL Gelfiltration als ein höher molekulares Produkt vor, welches ein Molekulargewicht von ≈ 250 kDa hat (Beispiel N). In Fig. M sind schematische Darstellungen des 431/26 hum V_{H} huβGlc1H Fusionsproteins gezeigt. Die Figur Mb zeigt das Monomer, welches eine ≈ 25 kDa leichte Kette und eine ≈ 100 kDa schwere Kette besitzt. Dieses Monomer und ein über "inter heavy chain" Disulfidbrücken verknüpftes Dimer läßt sich unter denaturierenden Bedingungen nachweisen (Fig. Ma). Unter nativen Bedingungen liegt das Fusionsprotein als Dimer von ≈ 250 kDa vor, mit bzw. ohne "inter heavy chain" Disulfidbrücken (Fig. Mc).

### BEISPIEL P:

### Chemische Modifizierung des Fusionsproteins

Das nach Beispiel N gereinigte Fusionsprotein (110 µg/ml) wurde auf pH 4.5 eingestellt und mit Natriumperiodat versetzt (Endkonzentration 1 mM). Nach 1stündiger Inkubation bei Raumtemperatur im Dunklen wurde das Natriumperiodat mittels Gelchromatographie entfernt und das Fusionsprotein dann auf pH 8 umgestellt. Nach Zugabe von Ethanolamin bis zu einer Endkonzentration von 0.1 M wurde weitere 3 Stunden bei 4° C inkubiert, dann Natriumcyanoborhydrid (Endkonzentration 5 mM) dazugegeben und 30 min inkubiert (Reduktion). Daran schloß sich wieder eine Gelfiltration zur Entfernung des Reduktionsmittels und zur Umpufferung des Fusionsproteins an. Die chemische Modifikation hatte keine Auswirkung auf die funktionelle Aktivität des Fusionsproteins. Tab. 7 zeigt einen Verlauf der Plasmakonzentrationen von unmodifiziertem und modifiziertem Fusionsprotein in der Nacktmaus. Die Eliminierung des Fusionsproteins aus dem Plasma ist durch die Modifizierung stark verlangsamt.

**Tabelle 7**

| **Plasmaspiegel der β-Glucuronidaseaktivität in der Nacktmaus** | | |
|---|---|---|
| t [min] | β-Glucuronidase-Fusionsprotein | |
| | behandelt % Aktivität | unbehandelt % Aktivität |
| 0 | 100 | 100 |
| 10 | | 54 |
| 30 | 76 | |
| 60 | | 22 |
| 240 | 40 | 4 |
| 480 | | 1 |
| 1380 | 19 | |
| 1440 | | 1 |
| 5880 | 3 | |

### BEISPIEL Q:

### Enzymatische Behandlung des Fusionsproteins

53 µg Fusionsprotein (Beispiel N) in 0.01 M Tris/HCl, 0.15 M NaCl wurden mit 1 Unit löslicher alkalischer Phosphatase (E. coli) bzw. immobilisierter alkalischer Phosphatase (bovine intestine) 20 h bei Raumtemperatur inkubiert. Tab. 8 zeigt den Verlauf der Plasmakonzentration von unbehandeltem und behandeltem Fusionsprotein in der Nacktmaus. Die Eliminierung wird durch die Enzymbehandlung nicht signifikant beeinflußt.

**Tabelle 8**

| **Plasmaspiegel der β-Glucuronidaseaktivität in der Nacktmaus** | | | |
|---|---|---|---|
| t [min] | β-Glucuronidase Fusionsprotein | | |
| | unbehandelt [%] | AP (Rinderdarm,immobil.) behandelt [%] | AP (E. coli) behandelt [%] |
| 0 | 100 | 100 | 100 |
| 10 | 78 | 76 | 65 |
| 30 | 44 | 57 | 53 |
| 60 | 35 | 36 | 35 |
| 240 | 22 | 27 | 22 |
| 1440 | 4 | 5 | 5 |
| AP = alkalische Phosphatase | | | |

### Beispiel R:

### Pharmakokinetik und Tumorretention des 431/26 hum V_{H} huβGlc 1H Fusionsproteins

Beispielhaft wurden 5x 4 µg gereinigtes und mit 100 µg HSA/ml vermischtes Fusionsprotein in unmodifizierter (Beispiel N) bzw. chemisch modifizierter Form (Beispiel P) i.v. in 24stündigen Abständen in CEA exprimierende humane Tumor-tragende Nacktmäuse injiziert. Nach definierten Zeiträumen wurden je 3 Tiere durch Genickbruch getötet. Die Organe wurden entnommen, gewogen und mit 2 ml 1%igem BSA in PBS, pH 7.2, versetzt. Anschliessend wurden die Gewebe und Zellen aus diesen Organen durch pottern zerstört (10 Stöße) und die Menge an funktionell aktivem Fusionsprotein nach Zentrifugation der Suspension bei 3000 rpm und RT für 10' (Heraeus Labofuge GL, Typ 2202) im Spezifitätsenzym-Aktivitätstest (siehe Beispiel J) in Überstand bestimmt. Die Daten aus einem repräsentativen Experiment sind in Tab. 9 dargestellt. Es ist deutlich zu erkennen, daß sich das chemisch modifizierte Fusionsprotein, welches eine t1/2β von ≈ 4 h hat, spezifisch ab ≥ 3 Tagen nach Beendigung der repetitiven Injektionsphase im Tumor anreichert. Das unmodifizierte Fusionsprotein, welches eine t1/2β von ≈ 20 min besitzt, zeigte unter den gleichen experimentellen Bedingungen keine signifikante Tumoranreicherung.

Aus diesen Daten darf gefolgert werden, daß das hu 431 β-Gluc Fusionsprotein in der Lage ist, in vivo an CEA-positive Tumore zu binden und dort als enzymatisch aktives Molekül über lange Zeiträume (> 9 Tage) zu verweilen. Der Zeitpunkt für die Anwendung der Prodrug sollte in diesem System zwischen Tag 3 und 9 nach Beendigung der Fusionsproteininjektion liegen.

**Tabelle 9**

| Tumorretentionsexperiment mit CEA-positivem humanem Magenkarzinom Xenograft (Mz Sto 1) | | | | | | | |
|---|---|---|---|---|---|---|---|
| i.v. Injektion von 5 x 4 µg Fusionsprotein pro Maus in 24-Stundenintervallen | | | | | | | |

| Zelt (Std.) nach letzter i.v. Injektion | % Aktivität/g Tumor oder Organ | | | | | | |
|---|---|---|---|---|---|---|---|
| | Tumor | Leber | Lunge | Milz | Niere | Eingeweide | Plasma |
| 3 | 100 | 100 | 100 | 100 | 100 | 100 | 100 |
| 24 | 86.7 | 49.3 | 26.3 | - | 64 | 123 | 44.4 |
| 72 | 26.4 | 11 | 4.4 | - | 8.1 | 19 | 6.5 |
| 216 | 24.4 | 1.5 | 0.13 | 1.2 | 0.4 | 0.5 | 0.015 |

### Beispiel S:

### Isoelektrische Fokussierung des 431/26 hum V_{H} hu βGlc 1H Fusionsproteins

Das mittels Anti-idiotyp Affinitätschromatographie gereinigte Fusionsprotein (Beispiel N) wurde im "Pharmacia Fast System" isoelektrisch entsprechend der Methode von Righetti et al. (1979) fokussiert. Hierbei zeigte sich, daß der isoelektrische Punkt des Moleküls in einem pH-Bereich von 7.35 bis 8.15 liegt.

### BEISPIEL T:

### Nachweis der Spaltbarkeit einer Zytostatika-Prodrug durch 431/26 hum V_{H} huβGlc 1H Fusionsprotein

Im Beispiel J wurde gezeigt, daß der β-Glucuronidaseanteil des Fusionsproteins, ähnlich wie das menschliche native Enzym, in der Lage ist, das β-Glucuronid des 4-Methylumbelliferol zu spalten. In den nachfolgend beschriebenen Untersuchungen wurde als Substrat für die enzymatische Spaltung ein über eine Spacergruppe verknüpftes β-Glucuronid des Zytostatikums Daunomycin eingesetzt. Im einzelnen wurden diese Untersuchungen wie folgt durchgeführt:
4 mg der in der französischen Patentanmeldung (No. d'Enregistrement National: 9105326) beschriebenen Verbindung N-(4-hydroxy-3-nitro-benzyloxycarbonyl)-daunorubicin-β-D-glucuronid (Prodrug) wurden in 1 ml 20 mM Phosphat-Puffer, pH 7.2, gelöst. Zu jeweils 5 µl dieser Substratlösung wurden 35 µl des Fusionsproteins (Beispiel N) bzw. der humanen β-Glucuronidase (Gesamtkonzentration jeweils 6.5 U/ml; 1 U = Spaltung von 1 µMol 4-Methylumbelliferol/min bei 37° C) hinzupipettiert und bei 37° C im Dunkeln inkubiert. Nach verschiedenen Zeiten wurden Proben (5 µl) des Inkubationsansatzes entnommen und direkt mittels Hochdruckflüssigkeitschromatographie unter folgenden Bedingungen analysiert:
Säule:
   Nucleosil 100 RP 18, 5 um Teilchendurchmesser, 125 x 4.6 mm
Mobile Phase:
   Gradient aus Lösung A (100 % Acetonitril) und Lösung B (20 mM Phosphat-Puffer pH 3.0)
0 min: 30 % Lösung A
15 min: 70 % Lösung A
20 min: 70 % Lösung A
Flußrate: 1 ml/min
Detektion: Fluoreszenz, Excitation 495 nm, Emission 560 nm
Datenauswertung: Beckman System Gold Software

Die Retentionszeit der Ausgangsverbindung (Prodrug) betrug unter diesen Chromatographiebedingungen 11 min. Die während der Inkubation entstehende Verbindung (Drug) wies eine mit Daunomycin (DNM, Analyse eines Standards unter gleichen Bedingungen) identische Retentionszeit von 8.9 min auf. Die Kinetik der Spaltung der Ausgangsverbindung durch das Fusionsprotein bzw. humane β-Glucuronidase sind in Tab. 11 bzw. Tab. 10 dargestellt.

Die Halbwertszeit der Spaltung der Prodrug durch das Fusionsprotein betrug 2.3 h. Die Spaltung durch humane β-Glucuronidase erfolgte mit einer Halbwertszeit von 0.8 h. Wie bereits im Beispiel J dargestellt zeigen die Ergebnisse der Untersuchungen, daß der β-Glucuronidaseanteil des Fusionsproteins funktionell aktiv und in der Lage ist, β-Glucuronide zu spalten. Die Kinetik der Abspaltung des Glucuronidanteiles bzw. die Freisetzung der Drug (Daunomycin) aus der verwendeten Prodrug erfolgt mit einer zu humaner β-Glucuronidase größenordnungsmäßig vergleichbaren Geschwindigkeit, so daß die Substratspezifität des Fusionsproteins im wesentlichen mit der von humaner β-Glucuronidase übereinstimmt.

**Tabelle 10**

| Kinetik der Prodrugspaltung durch β-Glucuronidase (human, rekombinant) | | |
|---|---|---|
| t min | prodrug area % | DNM area % |
| 0 | 99.2 | 0.8 |
| 57 | 36.0 | 64.0 |
| 130 | 10.3 | 89.7 |
| 227 | 9.3 | 90.7 |

**Tabelle 11**

| Kinetik der Prodrugspaltung durch β-Glucuronidase (Fusionsprotein) | | |
|---|---|---|
| t min | prodrug area % | DNM area % |
| 0 | 98.9 | 1.1 |
| 50 | 81.1 | 18.9 |
| 135 | 51.7 | 48.3 |
| 190 | 33.0 | 67.0 |
| 247 | 22.0 | 78.0 |
| 317 | 12.4 | 87.6 |

## Patentansprüche (Patentansprüche für folgende(n) Vertragsstaat(en): AT, BE, CH, LI, DE, DK, FR, GB, IT, LU, NL, SE)

1. Fusionsprotein zur Prodrug-Aktivierung der Formel huTuMAK-L-β-Gluc, wobei huTuMAK ein tumorbindendes Fragment eines humanisierten tumorspezifischen monoklonalen Antikörpers, welches Teile der schweren Kette enthält, die mit der leichten Kette des Antikörpers assoziiert sind, bedeutet, L für einen Peptid-Linker steht und β-Gluc humane β-Glucuronidase ist und in dem das Antikörpertragment aus huTuMAK Regionen besteht, welche von einem V_{H}-Exon und mindestens einem Hinge-Exon kodiert werden, und der Peptid-Linker L eine Region enthält, die von einem Hinge-Exon kodiert wird.

2. Fusionsprotein nach Anspruch 1, in dem das Antikörperfragment aus huTuMAK Regionen besteht, welche von einem V_{H}-Exon, einem CH₁-Exon und zwei Hinge-Exons kodiert werden.

3. Fusionsprotein nach einem der Ansprüche 1 bis 2, wobei das Antikörperfragment huTuMAK aus Aminosäuresequenzen gemäß Tab. 3 besteht.

4. Fusionsprotein nach einem der Ansprüche 1 bis 3, in dem L einen Peptid-Linker bedeutet, der von einer DNA gemäß Tab. 1 und Tab. 2 kodiert wird.

5. Fusionsprotein nach einem der Ansprüche 1 bis 4, enthaltend 2 oder 3 Hinge-Regionen eines humanen IgG3 C-Gens.

6. Plasmid, welches eine cDNA, die für ein Fusionsprotein gemäß einem der Ansprüche 1 bis 5 kodiert, enthält.

7. Transformierte eukaryotische Zelle, die mit einem Plasmid gemäß Anspruch 6 transformiert ist.

8. Verfahren zur Herstellung von einem Fusionsprotein gemäß einem der Ansprüche 1 bis 5, dadurch gekennzeichnet, daß in einer transformierten Zelle mittels eines Plasmids das Fusionsprotein exprimiert wird und die Isolierung dieses Fusionsproteins über antiidiotypische monoklonale Antikörper erfolgt.

9. Fusionsprotein, erhältlich durch Expression eines Fusionsproteins gemäß einem der Ansprüche 1 bis 5 in einer transformierten eukaryotischen Zelle gemäß Anspruch 7 und nachfolgender Behandlung des isolierten Fusionsproteins mit einem Oxidationsmittel.

10. Fusionsprotein, erhältlich durch reduktive Aminierung eines Fusionsproteins gemäß Anspruch 9.

11. Fusionsprotein gemäß einem der Ansprüche 1 bis 5 und 9 bis 10 zur Verwendung als Arzneimittel.

12. Verwendung eines Fusionsproteins gemäß einem der Ansprüche 1 bis 5 und 9 bis 10 zur Herstellung eines Arzneimittels, welches zur Prodrug-Aktivierung bei Tumorerkrankungen geeignet ist.

## Patentansprüche (Patentansprüche für folgende(n) Vertragsstaat(en): GR, ES)

1. Verfahren zur Herstellung eines Fusionsproteins zur Prodrug-Aktivierung der Formel huTuMAK-L-β-Gluc, wobei huTuMAK ein tumorbindendes Fragment eines humanisierten tumorspezifischen monoklonalen Antikörpers, welches Teile der schweren Kette enthält, die mit der leichten Kette des Antikörpers assoziiert sind, bedeutet, welcher über einen Peptid-Linker L mit humaner β-Glucuronidase β-Gluc verbunden ist, und in dem das Antikörperfragment aus huTuMAK Regionen besteht, welche von einem V_{H}-Exon, einem CH₁-Exon und mindestens einem Hinge-Exon kodiert werden, und der Peptid-Linker L eine Region enthält, die von einem Hinge-Exon kodiert wird, dadurch gekennzeichnet, daß in transformierten Zellen mittels Plasmiden das Fusionsprotein exprimiert wird.

2. Verfahren nach Anspruch 1, wobei das Antikörperfragment huTuMAK aus Fusionsprotein Regionen besteht, welche von einem V_{H}-Exon, einem CH₁-Exon und zwei Hinge-Exons kodiert werden.

3. Verfahren nach Anspruch 1 bis 2, dadurch gekennzeichnet, daß das Antikörperfragment huTuMAK des Fusionsproteins aus Aminosäuresequenzen gemäß Tab. 3 besteht.

4. Verfahren nach einem der Ansprüche 1 bis 3, dadurch gekennzeichnet, daß L des Fusionsproteins einen Peptid-Linker bedeutet, der von einer DNA gemäß Tab. 1 und Tab. 2 kodiert wird.

5. Verfahren nach einem der Ansprüche 1 bis 4, dadurch gekennzeichnet, daß das Fusionsprotein 2 oder 3 Hinge-Regionen eines humanen IgG3 C-Gens enthält.

6. Verfahren nach einem der Ansprüche 1 bis 5, dadurch gekennzeichnet, daß die Isolierung des Fusionsproteins mittels antiidiotypischer monoklonaler Antikörper erfolgt.

7. Verfahren zur Herstellung eines Plasmids, welches für ein Fusionsprotein gemäß einem der Ansprüche 1 bis 6 kodiert, dadurch gekennzeichnet, daß eine für das Fusionsprotein gemäß einem der Ansprüche 1 bis 6 kodierende cDNA in ein Plasmid kloniert wird.

8. Verfahren zur Herstellung einer transformierten eukaryotischen Zelle, dadurch gekennzeichnet, daß diese mit einem Plasmid gemäß Anspruch 7 transformiert wird.

9. Verfahren zur Herstellung eines Fusionsproteins gemäß einem der Ansprüche 1 bis 6, ferner dadurch gekennzeichnet, daß das experimentelle Fusionsprotein mit einem Oxidationsmittel behandelt wird.

10. Verfahren nach Anspruch 9, dadurch gekennzeichnet, daß in einem zweiten Reaktionsschritt das Fusionsprotein reduktiv aminiert wird.

11. Verfahren zur Herstellung eines Arzneimittels, dadurch gekennzeichnet, daß ein Fusionsprotein gemäß einem der Ansprüche 1 bis 6 und Ansprüche 9 bis 10 verwendet wird.

## Patentansprüche (Patentansprüche für folgende(n) Vertragsstaat(en): PT)

1. Fusionsprotein zur Prodrug-Aktivierung der Formel huTuMAK-L-β-Gluc, wobei HuTuMAK einen humanisierten tumorspezifischen monoklonalen Antikörper oder ein tumorbindendes Fragment davon, welches aus Teilen der schweren Kette besteht, die mit der leichten Kette des Antikörpers assoziiert sind, bedeutet, L für einen Peptid-Linker steht und β-Gluc humane β-Glucuronidase ist.

2. Fusionsprotein nach Anspruch 1, in dem das Antikörperfragment huTuMAK aus Regionen besteht, welche von einem V_{H}-Exon sowie einem CH₁₋Exon kodiert werden, und der Peptid-Linker L eine Region enthält, die von einem Hinge-Exon kodiert wird.

3. Fusionsprotein nach Anspruch 1 oder 2, in dem das Antikörperfragment huTuMAK aus Regionen besteht, welche von einem V_{H}-Exon, einem CH₁-Exon und einem Hinge-Exon kodiert werden.

4. Fusionsprotein nach Anspruch 1 oder 2, in dem das Antikörperfragment aus huTuMAK Regionen besteht, welche von einem V_{H}-Exon, einem CH₁-Exon und zwei Hinge-Exons kodiert werden.

5. Fusionsprotein nach einem der Ansprüche 1 bis 4, wobei das Antikörperfragment huTuMAK aus Aminosäuresequenzen gemäß Tab. 3 besteht.

6. Fusionsprotein nach einem der Ansprüche 1 bis 5, in dem L einen Peptid-Linker bedeutet, der von einer DNA gemäß Tab. 1 und/oder Tab. 2 kodiert wird.

7. Fusionsprotein nach einem der Ansprüche 1 bis 6, enthaltend 1,2 oder 3 Hinge-Regionen eines humanen IgG3 C-Gens.

8. Plasmid, welches eine cDNA, die für ein Fusionsprotein gemäß einem der Ansprüche 1 bis 7 kodiert, enthält.

9. Transformierte eukaryotische Zelle, die mit einem Plasmid gemäß Anspruch 8 transformiert ist.

10. Verfahren zur Herstellung von einem Fusionsprotein gemäß einem der Ansprüche 1 bis 7, dadurch gekennzeichnet, daß in einer transformierten Zelle mittels eines Plasmids das Fusionsprotein exprimiert wird und die Isolierung dieses Fusionsproteins über antiidiotypische monoklonale Antikörper erfolgt.

11. Fusionsprotein, erhältlich durch Expression eines Fusionsproteins gemäß einem der Ansprüche 1 bis 7 in einer transformierten eukaryotischen Zelle gemäß Anspruch 9 und nachfolgender Behandlung des isolierten Fusionsproteins mit einem Oxidationsmittel.

12. Fusionsprotein, erhältlich durch reduktive Aminierung eines Fusionsproteins gemäß Anspruch 11.

13. Fusionsprotein gemäß einem der Ansprüche 1 bis 7 und 11 bis 12 zur Verwendung als Arzneimittel.

14. Verwendung eines Fusionsproteins gemäß einem der Ansprüche 1 bis 7 und 11 bis 12 zur Herstellung eines Arzneimittels, welches zur Prodrug-Aktivierung bei Tumorerkrankungen geeignet ist.

## Claims (Claims for the following Contracting State(s): AT, BE, CH, LI, DE, DK, FR, GB, IT, LU, NL, SE)

1. A fusion protein for prodrug activation of the formula huTuMAb-L-β-Gluc, where huTuMAb is a tumor-binding fragment of a humanized tumor-specific monoclonal antibody, which fragment contains parts of the heavy chain which are associated with the light chain of the antibody, L is a peptide linker, and β-Gluc is human β-glucuronidase, and in which the antibody fragment huTuMAb consists of regions encoded by a V_{H} exon and at least one hinge exon, and the peptide linker L contains a region encoded by a hinge exon.

2. A fusion protein as claimed in claim 1, in which the antibody fragment huTuMAb consists of regions encoded by a V_{H} exon, a CH₁ exon and two hinge exons.

3. A fusion protein as claimed in either of claims 1 to 2, where the antibody fragment huTuMAb consists of amino acid sequences as shown in Tab. 3.

4. A fusion protein as claimed in any of claims 1 to 3, in which L is a peptide linker encoded by a DNA as shown in Tab. 1 and Tab. 2.

5. A fusion protein as claimed in any of claims 1 to 4, containing 2 or 3 hinge regions of a human IgG3 C gene.

6. A plasmid which contains a cDNA coding for a fusion protein as claimed in any of claims 1 to 5.

7. A transformed eukaryotic cell which is transformed with a plasmid as claimed in claim 6.

8. A process for the preparation of a fusion protein as claimed in any of claims 1 to 5, which comprises expressing the fusion protein in a transformed cell by means of a plasmid, and isolating this fusion protein via anti-idiotype monoclonal antibodies.

9. A fusion protein obtainable by expression of a fusion protein as claimed in any of claims 1 to 5 in a transformed eukaryotic cell as claimed in claim 7 and subsequently treating the isolated fusion protein with an oxidizing agent.

10. A fusion protein obtainable by reductive amination of a fusion protein as claimed in claim 9.

11. A fusion protein as claimed in any of claims 1 to 5 and 9 to 10 for use as pharmaceutical.

12. The use of a fusion protein as claimed in any of claims 1 to 5 and 9 to 10 for producing a pharmaceutical suitable for prodrug activation for oncoses.

## Claims (Claims for the following Contracting State(s): GR, ES)

1. A process for the preparation of a fusion protein for prodrug activation of the formula huTuMAb-L-β-Gluc, where huTuMAb is a tumor-binding fragment of a humanized tumor-specific monoclonal antibody, which fragment contains parts of the heavy chain which are associated with the light chain of the antibody which is linked via a peptide linker L to human β-glucuronidase β-Gluc, and in which the antibody fragment huTuMAb consists of regions encoded by a V_{H} exon, a CH₁ exon and at least one hinge exon, and the peptide linker L contains a region encoded by a hinge exon, which comprises expressing the fusion protein in transformed cells by means of plasmids.

2. The process as claimed in claim 1, where the antibody fragment huTuMAb consists of regions encoded by a V_{H} exon, a CH₁ exon and two hinge exons.

3. The process as claimed in claim 1 to 2, wherein the antibody fragment huTuMAb of the fusion protein consists of amino acid sequences as shown in Tab. 3.

4. The process as claimed in any of claims 1 to 3, wherein L in the fusion protein is a peptide linker encoded by a DNA as shown in Tab. 1 and Tab. 2.

5. The process as claimed in any of claims 1 to 4, wherein the fusion protein contains 2 or 3 hinge regions of a human IgG3 C gene.

6. The process as claimed in any of claims 1 to 5, wherein the fusion protein is isolated via anti-idiotype monoclonal antibodies.

7. A process for the preparation of a plasmid which codes for a fusion protein as claimed in any of claims 1 to 6, which comprises cloning a cDNA coding for the fusion protein as claimed in any of claims 1 to 6 into a plasmid.

8. A process for the preparation of a transformed eukaryotic cell, which comprises transforming it with a plasmid as claimed in claim 7.

9. A process for the preparation of a fusion protein as claimed in any of claims 1 to 6, which further comprises treating the experimental fusion protein with an oxidizing agent.

10. The process as claimed in claim 9, wherein the fusion protein is reductively aminated in a second reaction step.

11. A process for the production of a pharmaceutical, which comprises using a fusion protein as claimed in any of claims 1 to 6 and claims 9 to 10.

## Claims (Claims for the following Contracting State(s): PT)

1. A fusion protein for prodrug activation of the formula huTuMAb-L-β-Gluc, where huTuMAb is a humanized tumor-specific monoclonal antibody or a tumor-binding fragment thereof which consists of parts of the heavy chain which are associated with the light chain of the antibody, L is a peptide linker, and β-Gluc is human β-glucuronidase.

2. A fusion protein as claimed in claim 1, in which the antibody fragment huTuMAb consists of regions encoded by a V_{H} exon and by a CH₁ exon, and the peptide linker L contains a region encoded by a hinge exon.

3. A fusion protein as claimed in claim 1 or 2, in which the antibody fragment huTuMAb consists of regions encoded by a V_{H} exon, a CH₁ exon and a hinge exon.

4. A fusion protein as claimed in claim 1 or 2, in which the antibody fragment huTuMAb consists of regions encoded by a V_{H} exon, a CH₁ exon and two hinge exons.

5. A fusion protein as claimed in any of claims 1 to 4, where the antibody fragment huTuMAb consists of amino acid sequences as shown in Tab. 3.

6. A fusion protein as claimed in any of claims 1 to 5, in which L is a peptide linker encoded by a DNA as shown in Tab. 1 and/or Tab. 2.

7. A fusion protein as claimed in any of claims 1 to 6, containing 1, 2 or 3 hinge regions of a human IgG3 C gene.

8. A plasmid which contains a cDNA coding for a fusion protein as claimed in any of claims 1 to 7.

9. A transformed eukaryotic cell which is transformed with a plasmid as claimed in claim 8.

10. A process for the preparation of a fusion protein as claimed in any of claims 1 to 7, which comprises expressing the fusion protein in a transformed cell by means of a plasmid, and isolating this fusion protein via anti-idiotype monoclonal antibodies.

11. A fusion protein obtainable by expression of a fusion protein as claimed in any of claims 1 to 7 in a transformed eukaryotic cell as claimed in claim 9 and subsequently treating the isolated fusion protein with an oxidizing agent.

12. A fusion protein obtainable by reductive amination of a fusion protein as claimed in claim 11.

13. A fusion protein as claimed in any of claims 1 to 7 and 11 to 12 for use as pharmaceutical.

14. The use of a fusion protein as claimed in any of claims 1 to 7 and 11 to 12 for producing a pharmaceutical suitable for prodrug activation for oncoses.

## Revendications (Revendications pour l'(les) Etat(s) contractant(s) suivant(s): AT, BE, CH, LI, DE, DK, FR, GB, IT, LU, NL, SE)

1. Protéine de fusion pour l'activation d'un précurseur de médicament, de formule huTuMAK-L-β-Gluc, huTuMAK représentant un fragment, se fixant à une tumeur, d'un anticorps monoclonal humanisé spécifique de tumeur, contenant des segments de la chaîne lourde qui sont associés à la chaîne légère de l'anticorps, L représentant un lieur peptidique et β-Gluc étant la β-glucuronidase humaine, et dans laquelle le fragment anticorps consiste en régions de huTuMAk, qui sont codées par un exon V_{H} et au moins un exon charnière, et le lieur peptidique L contient une région qui est codée par un exon charnière.

2. Protéine de fusion selon la revendication 1, dans laquelle le fragment anticorps consiste en régions de huTuMAK qui sont codées par un exon V_{H}, un exon CH₁ et deux exons charnières.

3. Protéine de fusion selon la revendication 1 ou 2, dans laquelle le fragment anticorps huTuMAK est constitué de séquences d'aminoacides selon le tableau 3.

4. Protéine de fusion selon l'une des revendications 1 à 3, dans laquelle L représente un lieur peptidique qui est codé par un ADN selon le tableau 1 et le tableau 2.

5. Protéine de fusion selon l'une des revendications 1 à 4, contenant deux ou trois régions charnière d'un gène C d'IgG3 humaine.

6. Plasmide, qui contient un ADNc qui code pour une protéine de fusion selon l'une des revendications 1 à 5.

7. Cellule eucaryote transformée, qui est transformée par un plasmide selon la revendication 6.

8. Procédé pour la production d'une protéine de fusion selon l'une des revendications 1 à 5, caractérisé en ce que la protéine de fusion est exprimée au moyen d'un plasmide dans une cellule transformée, et l'isolement de cette protéine de fusion s'effectue au moyen d'anticorps monoclonaux anti-idiotypiques.

9. Protéine de fusion, pouvant être obtenue par expression d'une protéine de fusion selon l'une des revendications 1 à 5, dans une cellule eucaryote transformée, selon la revendication 7, et traitement subséquent par un oxydant de la protéine de fusion isolée.

10. Protéine de fusion, pouvant être obtenue par amination réductrice d'une protéine de fusion selon la revendication 9.

11. Protéine de fusion selon l'une des revendications 1 à 5 et 9 à 10, pour utilisation en tant que médicament.

12. Utilisation d'une protéine de fusion selon l'une des revendications 1 à 5 et 9 à 10, pour la fabrication d'un médicament qui est approprié à l'activation de précurseurs de médicaments, dans le cas de maladies tumorales.

## Revendications (Revendications pour l'(les) Etat(s) contractant(s) suivant(s): GR, ES)

1. Procédé pour la production d'une protéine de fusion pour l'activation d'un précurseur de médicament, de formule huTuMAK-L-β-Gluc, huTuMAK représentant un fragment, se fixant à une tumeur, d'un anticorps monoclonal humanisé spécifique de tumeur, contenant des segments de la chaîne lourde qui sont associés à la chaîne légère de l'anticorps, lequel fragment est lié, par un lieur peptidique L à la β-glucuronidase humaine β-Gluc, et dans laquelle le fragment anticorps consiste en régions de huTuMAK qui sont codées par un exon V_{H}, un exon CH₁ et au moins un exon charnière, et le lieur peptidique L contient une région qui est codée par un exon charnière, caractérisé en ce que la protéine de fusion est exprimée au moyen de plasmides dans des cellules transformées.

2. Procédé selon la revendication 1, dans lequel le fragment anticorps huTuMAK de la protéine de fusion consiste en régions qui sont codées par un exon V_{H}, un exon CH₁ et deux exons charnière.

3. Procédé selon la revendication 1 ou 2, caractérisé en ce que le fragment anticorps huTuMAK de la protéine de fusion est constitué de séquences d'aminoacides selon le tableau 3.

4. Procédé selon l'une des revendications 1 à 3, caractérisé en ce que L de la protéine de fusion représente un lieur peptidique qui est codé par un ADN selon le tableau 1 et le tableau 2.

5. Procédé selon l'une des revendications 1 à 4, caractérisé en ce que la protéine de fusion contient deux ou trois régions charnière d'un gène C d'IgG3 humaine.

6. Procédé selon l'une des revendications 1 à 5, caractérisé en ce que l'isolement de la protéine de fusion s'effectue au moyen d'anticorps monoclonaux anti-idiotypiques.

7. Procédé pour la production d'un plasmide qui code pour une protéine de fusion selon l'une des revendications 1 à 6, caractérisé en ce qu'un ADNc codant pour la protéine de fusion selon l'une des revendications 1 à 6 est cloné dans un plasmide.

8. Procédé pour la production d'une cellule eucaryote transformée, caractérisé en ce que celle-ci est transformée par un plasmide selon la revendication 7.

9. Procédé pour la production d'une protéine de fusion selon l'une des revendications 1 à 6, en outre caractérisé en ce que la protéine de fusion expérimentale est traitée par un oxydant.

10. Procédé selon la revendication 9, caractérisé en ce que, dans une seconde étape de réaction, la protéine de fusion est aminée par voie réductrice.

11. Procédé pour la fabrication d'un médicament, caractérisé en ce que l'on utilise une protéine de fusion selon l'une des revendications 1 à 6 et des revendications 9 et 10.

## Revendications (Revendications pour l'(les) Etat(s) contractant(s) suivant(s): PT)

1. Protéine de fusion pour l'activation d'un précurseur de médicament, de formule huTuMAK-L-β-Gluc, huTuMAK représentant un anticorps monoclonal humanisé spécifique de tumeur, ou un fragment de celui-ci se fixant à une tumeur, qui consiste en parties de la chaîne lourde qui sont associées à la chaîne légère de l'anticorps, L représente un lieur peptidique et β-Gluc est la β-glucuronidase humaine.

2. Protéine de fusion selon la revendication 1, dans laquelle le fragment anticorps huTuMAK consiste en régions qui sont codées par un exon V_{H} ainsi que par un exon CH₁, et le lieur peptidique L contient une région qui est codée par un exon charnière.

3. Protéine de fusion selon la revendication 1 ou 2, dans laquelle le fragment anticorps huTuMAK consiste en régions qui sont codées par un exon V_{H}, un exon CH₁ et un exon charnière.

4. Protéine de fusion selon la revendication 1 ou 2, dans laquelle le fragment anticorps consiste en régions huTuMAk qui sont codées par un exon V_{H}, un exon CH₁ et deux exons charnières.

5. Protéine de fusion selon l'une des revendications 1 à 4, dans laquelle le fragment anticorps huTuMAK consiste en séquences d'aminoacides selon le tableau 3.

6. Protéine de fusion selon l'une des revendications 1 à 5, dans laquelle L représente un lieur peptidique qui est codé par un ADN selon le tableau 1 et/ou le tableau 2.

7. Protéine de fusion selon l'une des revendications 1 à 6, contenant un, deux ou trois régions charnière d'un gène C d'IgG3 humaine.

8. Plasmide, qui contient un ADNc qui code pour une protéine de fusion selon l'une des revendications 1 à 7.

9. Cellule eucaryote transformée, qui est transformée par un plasmide selon la revendication 8.

10. Procédé pour la production d'une protéine de fusion selon l'une des revendications 1 à 7, caractérisé en ce que la protéine de fusion est exprimée au moyen d'un plasmide dans une cellule transformée, et l'isolement de cette protéine de fusion s'effectue au moyen d'anticorps monoclonaux anti-idiotypiques.

11. Protéine de fusion, pouvant être obtenue par expression d'une protéine de fusion selon l'une des revendications 1 à 7, dans une cellule eucaryote transformée, selon la revendication 9, et traitement subséquent par un oxydant de la protéine de fusion isolée.

12. Protéine de fusion, pouvant être obtenue par amination réductrice d'une protéine de fusion selon la revendication 11.

13. Protéine de fusion selon l'une des revendications 1 à 7 et 11 à 12, pour utilisation en tant que médicament.

14. Utilisation d'une protéine de fusion selon l'une des revendications 1 à 7 et 11 à 12, pour la fabrication d'un médicament qui est approprié à l'activation de précurseurs de médicaments, dans le cas de maladies tumorales.
